# NEUE EUROPÄISCHE PATENTSCHRIFT

(11) **EP 0 506 868 B2**
(45) Veröffentlichungstag und Bekanntmachung des Hinweises auf die Entscheidung über den Einspruch: **11.02.2004**
(45) Hinweis auf die Patenterteilung: 17.07.1996
(21) Anmeldenummer: 91902687.2
(22) Anmeldetag: 21.12.1990
(51) Int. Cl.: C07K 14/00, C12N 15/31, G01N 33/569, A61K 39/02

(54) **IMMUNOLOGISCH AKTIVE PROTEINE VON BORRELIA BURGDORFERI, ZUSAMMENHÄNGENDE TESTKITS UND IMPFSTOFF**
IMMUNOLOGICALLY ACTIVE PROTEINES FROM BORRELIA BURGDORFERI, RELATED TEST KITS AND VACCINE
PROTEINES IMMUNOLOGIQUEMENT ACTIVES DE BORRELIA BURGDORFERI, COFFRETS D'ANALYSE CONNEXES ET VACCINS

(30) Priorität: 22.12.1989 DE 3942728; 13.06.1990 DE 4018988
(43) Veröffentlichungstag der Anmeldung: 07.10.1992
(73) Patentinhaber: MIKROGEN MOLEKULARBIOLOGISCHE ENTWICKLUNGS-GMBH, D-80339 München (DE)
(72) Erfinder: FUCHS, Renate, D-8024 Deisenhofen (DE); WILSKE, Bettina, D-8000 München 90 (DE); PREAC-MURSIC, Vera, D-8000 München 40 (DE); MOTZ, Manfred, D-8000 München 70 (DE); SOUTSCHEK, Erwin, D-8000 München 70 (DE)
(74) Vertreter: Keller, Günter, Dr.
(86) Internationale Anmeldenummer: PCT/EP1990/002282
(87) Internationale Veröffentlichungsnummer: WO 1991/009870

(56) Entgegenhaltungen:
- NUCLEIC ACIDS RESEARCH, Band 17, Nr. 9, 11. Mai 1989, IRL Press Plc.; G.S. GASSMANN et al., Seite 3590
- NUCLEIC ACIDS RESEARCH, Band 17, Nr. 21, 11. November 1989, IRL Press Plc.; R. WALLICH et al., p. 8864
- PROCEEDINGS OF THE NATL. ACADEMY OF SCIENCES USA, Band 87, Mai 1990 Washington, DC (US); U.E. SCHAIBLE et al., Seiten 3768-3772
- ANNALS of the New York Academy of Science, vol. 539, 1988; p. 126
- INFECTION & IMMUNITY, vol. 57, 1989; p. 3637
- Gassman et al., Nucleic Acids Research, 1989
- Gassmann et al., FEMS Microbiologie Letters, 1989
- Hansen et al., Journal of Clinical Microbiology, 1988
- Maniatis et al., Molecular Cloning, 1989
- Lee et al., Science,vol 239, pp 1288-1291
- Pallesen et al., Infection and Immunity, 1989
- Wallich, et al., Infection and Immunity, 1990
- Coleman et al., J. Clin. Invest., 1989
- Luft et al., Infection and Immunity, 1989
- Wilske et al., Annals New York Acad., 1988
- Wilske et al., Zbl. Bakt. Suppl.18, 1989.
- Harlow et al., Antibodies, 1988
- Fuchs et al., Molecular Mikrobiology, 1992
- Preac-Mursic et al., Infection 20, 1992
- Barbour, Journal of Clinical Microbiology, 1988, pp475-478
- Sadziene et al., Infection and Immunity, vol. 61 No.5, 1993
- Jauris-Heipke et al., Med, Microbiol. Immunol., 1993, 182:37-50
- Wilske et al., Med. Microbiol. Immunol., 1994, 183:43-59
- Geysen et al., J. Immunol. Methods, 1987, 102:259-274
- Geysen et al., Proc. Natl. Acad. Sci., 1984, vol.81 pp.3998-4002

## Beschreibung

Die Lyme-Borreliose ist in der Bundesrepublik Deutschland die häufigste von Zecken übertragene Infektionskrankheit des Menschen. Im Gegensatz zur Frühsommer-Meningoencephalitis (FSME), die ebenfalls durch Zecken übertragen wird, beschränkt sich die Lyme-Borreliose nicht auf wenige Endemiegebiete, sondern kommt in allen Ländern der BRD vor. Die Durchseuchung des Hauptvektors in Europa, Ixodes ricinus, mit dem Erreger der Lyme-Borreliose, der Spirochäte Borrelia burgdorferi, liegt im süddeutschen Raum bei ca. 20 % der Adulten, ca. 10 % der Nymphen und bei ca. 1 % der Larven. Der Hauptvektor in USA, Ixodes dammini, kann in Hochendemiegebieten bis zu 100 % mit Borrelien befallen sein.

B. burgdorferi gehört zur Familie der Spirochäten. Spirochäten sind schraubenförmige Bakterien von 8-30 µm Länge. Sie bestehen aus einer äußeren Hülle, den Endoflagellen im Periplasma und dem Protoplasmazylinder. Der Protoplasmazylinder ist ein Komplex aus Cytoplasma, innerer Zellmembran und Peptidoglykan. Zu den human-pathogenen Vertretem der Spirochäten gehören neben B. burgdorferi die Rückfallfieberborrelien (z.B. B. recurrentis), der Erreger der Syphilis (Treponema (T.) pallidum) und die Leptospiren. Auf Grund der nahen immunologischen Verwandtschaft der Erreger sind Kreuzreaktionen ein Problem beim serologischen Nachweis von Antikörpern bei Syphilis und Lyme-Borreliose mit bisher verfügbaren Testen.

Eine Infektion mit B. burgdorferi führt zu einem komplexen Krankheitsbild, das man ähnlich wie bei der Syphilis, in drei verschiedene Stadien einteilen kann. Die wichtigsten Manifestationen sind:
- Frühphase: Stadium I: Erythema migrans
lymphozytäre Meningoradikulitis Bannwarth (LMR)
Borrelien-Lymphozytom
- Spätphase: Stadium III: Lymearthritis
Acrodermatitis chronica atrophicans (ACA)
chronische Borrelien-Encephalomyelitis

Seltenere klinische Manifestationen sind: Karditis, Myositis, Iritis und Panophthalmitis. Die diaplacentare Übertragung des Erregers ist möglich, jedoch sind bisher nur wenige Fälle einer connatalen Lyme Borreliose dokumentiert. Die verschiedenen Stadien können einzeln oder kombiniert auftreten. Die Infektion mit B. burgdorferi kann auch subklinisch verlaufen. Epidemiologische Studien an 375 klinisch nachgewiesenen Fällen zeigten einige Besonderheiten in der Alters- und Geschlechtsverteilung bei den verschiedenen klinischen Manifestationen. So fanden sich Patienten mit Erythema migrans am häufigsten in der Altersgruppe der 30-bis 60-jährigen. Neurologische Manifestationen zeigten zwei Altersgipfel: den ersten bei Kindern und Jugendlichen bis 20 Jahren, den zweiten bei 40- bis 70-jährigen. Die Lyme-Arthritis wurde am häufigsten bei 30- bis 60-jährigen beobachtet. Patienten mit ACA waren in keinem Falle jünger als 30 Jahre. Die ACA betrifft deutlich häufiger Frauen als Männer. Die serologische Untersuchung zeigte im Immunfluoreszenztest bei Patienten mit Erythema migrans überwiegend positive IgM-Befunde, bei neurologischen Manifestationen überwiegend positive IgG-Befunde. Bei den Spätmanifestationen ACA und Lyme-Arthritis waren die IgG-Titer regelmäßig erhöht und IgM-Antikörper nur mehr in Ausnahmefällen nachweisbar.

Für die Diagnostik steht sowohl der Erregernachweis als auch der Antikörpernachweis zur Verfügung. Der Erregernachweis aus Patientenmaterial (Hautbiopsien, Liquor, Punktate) ist besonders im Frühstadium (Erythema migrans), bei dem ein Antikörpernachweis häufig negativ ist, zu empfehlen. Allerdings ist zur Anzüchtung von B. burgdorferi ein komplexes Nährmedium nötig (Preac-Mursic, V.; Wilske, B.; Schierz, G. (1986): European Borreliae burgdorferi isolated from humans and ticks - culture conditions and antibiotic susceptibility. Zbl.Bakt.Hyg.A 163, 112-118) und die Kultivierung ist daher Speziallabors vorbehalten. Für die Isolierung des Erregers wird außerdem eine Zeit bis zu 5 Wochen benötigt. Die Isolierung von B. burgdorferi gelingt aus Hautproben in 50-70 % der Fälle mit Hautmanifestationen und in 3-5 % der Fälle mit Neuroborreliose (Preac-Mursic, V.; unpubl. Ergebnisse).

Der Antikörpernachweis (IgM, IgG) wird im Serum und bei neurologischen Manifestationen auch aus Liquor durchgeführt. Der serologische Befund ist abhängig vom Stadium der Erkrankung, der Dauer der Symptome und einer evt. schon erfolgten Antibiotika-Therapie. So ist der Antikörpernachweis mit bisher verfügbaren Testen beim Erythema migrans nur in 20-50 % der Fälle erfolgreich. bei neurologischen Manifestationen in 50-90 % und bei ACA und Arthritis in 90-100%.

Die Therapie der Lyme-Borreliose wird überwiegend mit Penicillin G, Tetracyclinen, Erythromycin oder Cephalosporinen durchgeführt. Die Lyme-Borreliose heilt zwar in den frühen Stadien oft spontan aus, allerdings sind auch dann Spätmanifestationen nicht ausgeschlossen. Daher ist eine Therapie im Frühstadium unerläßlich. Zudem ist eine klinische Heilung nach Antibiotikatherapie bei Spätmanifestationen nur in einem Teil der Fälle zu erzielen (z.B. nur ca. 50 % bei Lyme-Arthritis).

Daher sollte die Lyme Borreliose möglichst frühzeitig diagnostiziert werden. Da (wie bereits erläutert) die Erregerisolierung teuer, zeitaufwendig und zudem auch nicht immer erfolgreich ist, sollten bessere serodiagnostische Tests entwickelt werden. Die bisher benutzten Verfahren (Immunfluoreszenztest (IFT), indirekter Hämagglutinationstest (IHA), Enzyme-linked immunosorbent assay (ELISA) versagen oftmals in den Frühstadien. Als Antigene werden für diese Teste ganze B. burgdorferi Zellen oder Gesamtzell- Ultrasonikate eingesetzt. Die Verwendung unterschiedlicher B. burgdorferi Stämmen als Antigen führt im Ultrasonikat-ELISA zu unterschiedlichen Testergebnissen. Die Zellen werden auf Objektträger bzw. Ultrasonikat-Antigen auf Mikrotiterplatten fixiert, mit Serum oder Liquor inkubiert und die Borrelien-spezifischen Antikörper mit einem zweiten Fluoreszenz- oder Peroxidase- markierten Antikörper der entsprechenden Immunglobulinklasse detektiert. Die Reaktion wird dann entweder im Fluoreszenzmikroskop (IFT) oder nach einer Farbreaktion im Photometer (ELISA) ausgewertet.

Ein Problem für die Spezifität der Teste sind breite Kreuzreaktionen des Erregers B. burgdorferi zu anderen bakteriellen Erregern, insbesonder zu T pallidum, dem Erreger der Syphilis. Da die Testantigene im allgemeinen aus Lysaten des gesamten Erregers bestehen, werden auch Antikörper gegen sog. "common antigens' erfaßt (Hansen, K.; Hindersson, P.; Pedersen, N.S.(1988): Measurement of antibodies to the Borrelia burgdorferi flagellum improves serodiagnosis in Lyme disease. J.Clin.Microbiol., 26, 338-346). 'Common antigens" sind weit verbreitete und in ihrer Sequenz stark konservierte Proteine, d.h. die "Common antigens" von Borrelien, Treponemen, aber auch zahlreicher anderer Bakterien besitzen gemeinsame Epitope. Daneben könne falsch positive Reaktionen im IgM-IFT oder IgM-ELISA bei Seren mit Rheumafaktor-Aktivität auftreten. Um die Teste spezifischer zu machen, wird daher beim Nachweis von IgG- und IgM-Antikörpem eine Prae-Absorption der Seren mit einem Treponema-Ultrasonikat und zusätzlich beim Nachweis von IgM-Antikörpem noch eine Absorption mit Rheumafaktorabsorbens durchgeführt.

Gassmann et al. (Nucleic Acids Research, Vol. 17 (1989) S. 3590) beschreiben die DNA-Sequenz eines Hauptgeißelproteins aus dem Borrelia burgdorferi-Stamm B31.

Wallich et al. (Nucleic Acids Research, Vol. 17 (1989) S. 8864) beschreiben die Klonierung und Sequenzierung eines Gens; das für das Protein A der äußeren Oberfläche (OspA) des Borrelia burgdorferi-Stammes ZS7kodiert.

Wilske et al. (Annals New York Acad. Science, 1988, Vol. 539, S. 126-143) bechreiben die antigenetische Variabilität von verschiedenen Borrelia burgdorferi-Stämmen. Die Untersuchungen wurden im wesentlichen mit ganzen Borrelien oder mit Zellysaten von ganzen Zellen durchgeführt. Die Herstellung von einzelnen Proteinen mit Hilfe gentechnologischer Methoden wird in dieser Literaturstelle nicht beschrieben.

Luft et al. (Infection and Immunity (November 1989), S. 3637-3645) beschreiben die biochemische und immunologische Charakterisierung von Oberflächenproteinen aus Borrelia burgdorferi, wobei insbesondere der Stamm B31 verwendet wurde. Die in dieser Literaturstelle angegebene aminoterminale Sequenz des 22 kDA-Proteins unterscheidet sich völlig von der entsprechenden Sequenz des erfindungsgemäßen Proteins.

Aufgabe der vorliegenden Erfindung ist es daher immunologisch aktive Proteine von Borrelia burgdorferi zur Verfügung zu stellen, die in einem Testkit Verwendung finden, das die oben erwähnten Nachteile nicht aufweist. Weiterhin soll dieses Testkit den schnellen und zuverlässigen Nachweis von gegen Borrelia burgdorferi gerichteten Antikörpern ermöglichen.

Darüber hinaus sollen immunologisch aktive Proteine zur Verfügung gestellt werden, die sich als Impfstoffe gegen durch Borrelien-Stämme verursachte Infektionen eignen.

Bei der Untersuchung von Patientenseren aus unterschiedlichen Krankheitsstadien der Lyme-Borreliose im Western Blot sowie der Untersuchung von Nicht-Lyme-Borretiose-Patienten (insbesondere Syphilis-Patienten) auf Kreuzreaktivität mit B. burgdorferi wurden immunologisch aktive Proteine (B. burgdorferi-Antigene) gefunden, die einerseits eine gute Antikörper-Antwort nach Infektion hervorrufen und zum anderen eine geringe Kreuzreaktivität mit nicht B. burgdorferi-positiven Seren zeigen (Beispiel 1 ). Es zeigte sich, daß ein bestimmter Stamm von B. burgdorferi mit der laborinternen Kennzeichnung PKo der bei der Deutschen Sammlung für Mikroorganismen (DSM) unter der Nr. 5662 hinterlegt wurde, unter anderem ein immundominantes Protein im Molekulargewichtsbereich um etwa 22 kD (pC-Protein) besitzt. Die Bestimmung des Molekulargewichts der erfindungsgemäßen Proteine erfolgte nach an sich bekannten Methoden insbesondere durch SDS-Gelelektrophorese. Es wurde gefunden, daß dieses Protein immundominant für die IgM-Antwort ist. Dieses Protein ist nicht in allen B. burgdorferi-Stämmen in gleicher Weise ausgeprägt. Erfindungsgemäß wurde dieses immunologisch aktive Protein (pC-Protein) gentechnologisch hergestellt (Beisp. 3).

Auch andere immunologisch aktive Proteine (Antigene), die sich in besonderer Weise für die Verwendung in Testkits eignen, wurden in allgemein zugänglichen und kommerziell erhältlichen Escherichia coli-Zellen, wie beispielsweise den Stämmen JM 105 (Pharmacia) oder DH 5 (Gibco-BRL) hergestellt. Hierzu wurden die für diese Proteine kodierenden B. burgdorferi DNA-Fragmente isoliert und anschließend in effektive Expressionsvektoren eingesetzt (Beisp. 2 und 3).

Die Identifizierung und Isolierung der entsprechenden DNA-Fragmente erfolgte nach verschiedenen Methoden. So wurde ein nicht erfindungsgemaßes immunologisch aktives Protein mit einem Molekulargewicht von etwa 41 kD, das im folgenden auch als p41-Protein bezeichnet wird, mittels Polymerase chain reaction (PCR) und spezifischen Primem, deren Sequenzen synthetisch hergestellt wurden, dargestellt (Beisp. 2).

Weiterhin wurde eine Genbank des B. burgdorferi-Genoms erstellt, die mittels monoklonaler Antikörper auf die direkte Expression von immunologisch aktiven Proteinen durchsucht wurde.

In entsprechender Weise wurden auch Proteine mit Molekulargewichten von etwa 100 kD und 31 kD kloniert und sequenziert. Eine weitere Methode bestand darin, bestimmte ausgewählte immunologisch aktive Proteine (Antigene) aus B. burgdorferi-Lysaten zu reinigen und Aminosäuresequenzen dieser Antigene zu ermitteln. Anschließend wurden der Aminosäuresequenz entsprechende Oligodesoxynukleotide synthetisiert und durch Hybridisierung diejenigen Klone der Genbank identifiziert, die für die immunologisch aktiven Proteine kodierende DNA-Sequenzen aufwiesen. Die beiden letztgenannten Methoden werden im Beispiel 3 näher erläutert.

Nach Charakterisierung, Sequenzierung und Umklonierung der Gene in entsprechende Expressionsvektoren wurden die Antigene in E. coli-Zellen exprimiert und anschließend gereinigt. Ein Devorzugtes Reinigungsverfahren wird in Beispiel 4 beschrieben.

Die erfindungsgemäß hergestellten immunologisch aktiven Proteine von Borrelia burgdorferi können in Testkits verwendet werden, die einen überraschend sensitiven Nachweis von Antikörpern gegen B. burgdorferi in verschiedenen Untersuchungsflüssigkeiten zur Verfügung stellen. Ein Vorteil der erfindungsgemäß hergestellten immunologisch aktiven Proteine besteht darin, daß die Präparationen nur aus dem gewünschten Protein und möglicherweise solchen Proteinen bestehen, die auf Degradationserscheinungen und/oder unvollständige Translation zurückzuführen sind. Diese Präparationen enthalten keine solchen B. burgdorferi-Proteine, die nicht dem rekombinant erzeugten Protein entsprechen, da sie gentechnologisch hergestellt wurden.

Die erfindungsgemäßen Proteine sind dadurch gekennzeichnet, daß sie bzw. es
a) die Aminosäuresequenz 
Met Lys Lys Asn Thr Leu Thr Ala Ile Leu Met Thr Leu Phe Leu
Phe Ile Ser Cys Asn Asn Ser Gly Lys Val Gly Ile Leu Thr Ser
Thr Asn Pro Ala Asp Glu Ser Ala Lys Gly Pro Asn Leu Thr Glu
Ile Ser Lys Lys Ile Thr Asp Ser Asn Ala Phe Val Leu Ala Val
Lys Glu Val Glu Thr Leu Val Leu Ser Ile Asp Glu Leu Ala Lys
Lys Ala Ile Gly Gln Lys Ile Asp Asn Asn Asn Gly Leu Ala Ala
Leu Asn Asn Gln Asn Gly Ser Leu Leu Ala Gly Ala Tyr Ala Ile
Ser Thr Leu Ile Thr Glu Lys Leu Ser Lys Leu Lys Asn Leu Glu
Glu Leu Lys Thr Glu Ile Ala Lys Ala Lys Lys Cys Ser Glu Glu
Phe Thr Asn Lys Leu Lys Ser Gly His Ala Asp Leu Gly Lys Gln
Asp Ala Thr Asp Asp His Ala Lys Ala Ala Ile Leu Lys Thr His
Ala Thr Thr Asp Lys Gly Ala Lys Glu Phe Lys Asp Leu Phe Glu
Ser Val Glu Gly Leu Leu Lys Ala Ala Gln Val Ala Leu Thr Asn
Ser Val Lys Glu Leu Thr Ser Pro Val Val Ala Glu Ser Pro Lys
Lys Pro
 oder eine Teilsequenz davon von wenigstens 10 Aminosäuren aufweisen oder
b) die Aminosäuresequenz 
Met Lys Lys Asn Thr Leu Ser Ala Ile Leu Met Thr Leu Phe Leu
Phe Ile Ser Cys Asn Asn Ser Gly Lys Asp Gly Asn Thr Ser Ala
Asn Ser Ala Asp Glu Ser Val Lys Gly Pro Asn Leu Thr Glu Ile
Ser Lys Lys Ile Thr Asp Ser Asn Ala Val Leu Leu Ala Val Lys
Glu Val Glu Ala Leu Leu Ser Ser Ile Asp Glu Ile Ala Ala Lys
Ala Ile Gly Lys Lys Ile His Gln Asn Asn Gly Leu Asp Thr Glu
Asn Asn His Asn Gly Ser Leu Leu Ala Gly Ala Tyr Ala Ile Ser
Thr Leu Ile Lys Gln Lys Leu Asp Gly Leu Lys Asn Glu Gly Leu
Lys Glu Lys Ile Asp Ala Ala Lys Lys Cys Ser Glu Thr Phe Thr
Asn Lys Leu Lys Glu Lys His Thr Asp Leu Gly Lys Glu Gly Val
Thr Asp Ala Asp Ala Lys Glu Ala Ile Leu Lys Thr Asn Gly Thr
Lys Thr Lys Gly Ala Glu Glu Leu Gly Lys Leu Phe Glu Ser Val
Glu Val Leu Ser Lys Ala Ala Lys Glu Met Leu Ala Asn Ser Val
Lys Glu Leu Thr Ser Pro Val Val Ala Glu Ser Pro Lys Lys Pro
 oder eine Teilsequenz davon von wenigstens 10 Aminosäuren aufweisen oder
c) aus der Aminosäuresequenz 
Met Arg Gly Ser Ile Met Ile Ile Asn His Asn Thr Ser Ala Ile
Asn Ala Ser Arg Asn Asn Ala Ile Asn Ala Ala Asn Leu Ser Lys
Thr Gln Glu Lys Leu Ser Ser Asn Tyr Arg Ile Asn Arg Ala Ser
Asp Asp Ala Ala Gly Met Gly Val Ser Gly Lys Ile Asn Ala Gln
Ile Arg Gly Leu Ser Gln Ala Ser Arg Asn Thr Ser Lys Ala Ile
Asn Phe Ile Gln Thr Thr Glu Gly Asn Leu Asn Glu Val Glu Lys
Val Leu Val Arg Met Lys Glu Leu Ala Val Gln Ser Gly Asn Gly
Thr Tyr Ser Asp Ser Asp Arg Gly Ser Ile Gln Ile Glu Ile Glu
Gln Leu Thr Asp Glu Ile Asn Arg Ile Ala Asp Gln Ala Gln Tyr
Asn Gln Met His Met Leu Ser Asn Lys Ser Ala Ser Gln Asn Val
Lys Thr Ala Glu Glu Leu Gly Met Gln Pro Ala Lys Ile Asn Thr
Pro Ala Ser Leu Ser Gly Ser Gln Ala Ser Trp Thr Leu Arg Val
His Val Gly Ala Asn Gln Asp Glu Ala Ile Ala Val Asn Ile Tyr
Ser Ala Asn Val Ala Asn Leu Phe Ala Gly Glu Gly Ala Gln Ala
Ala Gln Ala Ala Pro Val Gln Glu Gly Ala Gln Glu Glu Gly Ala
Gln Gln Pro Thr Pro Ala Thr Ala Pro Thr Gln Gly Gly Val Asn
Ser Pro Val Asn Val Thr Thr Thr Val Asp Ala Asn Thr Ser Leu
Ala Lys Ile Glu Asn Ala Ile Arg Met Ile Ser Asp Gln Arg Ala
Asn Leu Gly Ala Phe Gln Asn Arg Leu Glu Ser Ile Lys Asn Ser
Thr Glu Tyr Ala Ile Glu Asn Leu Lys Ala Ser Tyr Ala Gln Ile
Lys Asp Ala Thr Met Thr Asp Glu Val Val Ala Ala Thr Thr Asn
Ser Ile Leu Thr Gln Ser Ala Met Ala Met Ile Ala Gln Ala Asn
Gln Val Pro Gln Tyr Val Leu Ser Leu Leu Arg
 der der Teilsequenz dieses Proteins mit einer Deletion von 20-25 Aminosäuren am N-Terminus des Proteins besteht, oder
d) die Aminosäuresequenz 
Met Lys Lys Met Leu Leu Ile Phe Ser Phe Phe Leu Val Phe Leu
Asn Gly Phe Pro Leu Asn Ala Arg Glu Val Asp Lys Glu Lys Leu
Lys Asp Phe Val Asn Met Asp Leu Glu Phe Val Asn Tyr Lys Gly
Pro Tyr Asp Ser Thr Asn Thr Tyr Glu Gln Ile Val Gly Ile Gly
Glu Phe Leu Ala Arg Pro Leu Ile Asn Ser Asn Ser Asn Ser Ser
Tyr Tyr Gly Lys Tyr Phe Val Asn Arg Phe Ile Asp Asp Gln Asp
Lys Lys Ala Ser Val Asp Ile Phe Ser Ile Gly Ser Lys Ser Glu
Leu Asp Ser Ile Leu Asn Leu Arg Arg Ile Leu Thr Gly Tyr Leu
Ile Lys Ser Phe Asp Tyr Glu Arg Ser Ser Ala Glu Leu Ile Ala
Lys Ala Ile Thr Ile Tyr Asn Ala Val Tyr Arg Gly Asp Leu Asp
Tyr Tyr Lys Glu Phe Tyr Ile Glu Ala Ser Leu Lys Ser Leu Thr
Lys Glu Asn Ala Gly Leu Ser Arg Val Tyr Ser Gln Trp Ala Gly
Lys Thr Gln Ile Phe Ile Pro Leu Lys Lys Asn Ile Leu Ser Gly
Asn Val Glu Ser Asp Ile Asp Ile Asp Ser Leu Val Thr Asp Lys
Val Val Ala Ala Leu Leu Ser Glu Asn Glu Ser Gly Val Asn Phe
Ala Arg Asp Ile Thr Asp Ile Gln Gly Glu Thr His Lys Ala Asp
Gln Asp Lys Ile Asp Ile Glu Leu Asp Asn Phe His Glu Ser Asp
Ser Asn Ile Thr Glu Thr Ile Glu Asn Leu Arg Asp Gln Leu Glu
Lys Ala Thr Asp Glu Glu His Lys Lys Glu Ile Glu Ser Gln Val
Asp Ala Lys Lys Lys Gln Lys Glu Glu Leu Asp Lys Lys Ala Ile
Asp Leu Asp Lys Ala Gln Gln Lys Leu Asp Phe Ala Glu Asp Asn
Leu Asp Ile Gln Arg Asp Thr Val Arg Glu Lys Leu Gln Glu Asn
Ile Asn Glu Thr Asn Lys Glu Lys Asn Leu Pro Lys Pro Gly Asp
Val Ser Ser Pro Lys Val Asp Lys Gln Leu Gln Ile Lys Glu Ser
Leu Glu Asp Leu Gln Glu Gln Leu Lys Glu Ala Ser Asp Glu Asn
Gln Lys Arg Glu Ile Glu Lys Gln Ile Glu Ile Lys Lys Asn Asp
Glu Glu Leu Phe Lys Asn Lys Asp His Lys Ala Leu Asp Leu Lys
Gln Glu Leu Asn Ser Lys Ala Ser Ser Lys Glu Lys Ile Glu Gly
Glu Glu Glu Asp Lys Glu Leu Asp Ser Lys Lys Asn Leu Glu Pro
Val Ser Glu Ala Asp Lys Val Asp Lys Ile Ser Lys Ser Asn Asn
Asn Glu Val Ser Lys Leu Ser Pro Leu Asp Glu Pro Ser Tyr Ser
Asp Ile Asp Ser Lys Glu Gly Val Asp Asn Lys Asp Val Asp Leu
Gln Lys Thr Lys Pro Gln Val Glu Ser Gln Pro Thr Ser Leu Asn
Glu Asp Leu Ile Asp Val Ser Ile Asp Ser Ser Asn Pro Val Phe
Leu Glu Val Ile Asp Pro Ile Thr Asn Leu Gly Thr Leu Gln Leu
Ile Asp Leu Asn Thr Gly Val Arg Leu Lys Glu Ser Ala Gln Gln
Gly Ile Gln Arg Tyr Gly Ile Tyr Glu Arg Glu Lys Asp Leu Val
Val Ile Lys Ile Asp Ser Gly Lys Ala Lys Leu Gln Ile Leu Asp
Lys Leu Glu Asn Leu Lys Val Ile Ser Glu Ser Asn Phe Glu Ile
Asn Lys Asn Ser Ser Leu Tyr Val Asp Ser Arg Met Ile Leu Val
Val Val Lys Asp Asp Ser Asn Ala Trp Arg Leu Ala Lys Phe Ser
Pro Lys Asn Leu Asp Glu Phe Ile Leu Ser Glu Asn Lys Ile Leu
Pro Phe Thr Ser Phe Ala Val Arg Lys Asn Phe Ile Tyr Leu Gln
Asp Glu Leu Lys Ser Leu Val Thr Leu Asp Val Asn Thr Leu Lys
Lys Val Lys
 oder eine Teilsequenz davon von wenigstens 10 Aminosäuren aufweisen.

Unter dem Begriff "Testkits" wird ein Satz von Testreagentien verstanden, der den Nachweis von bestimmten Antikörpern ermöglicht. Die den Testkits zugrundeliegenden Prinzipien wurden in "Immunoassays for the 80s" (1981) von A. Voller et al., erschienen bei MTP Press Ltd, Falcon House, Lancaster, England beschrieben. Die Testreagentien weisen als wichtigste Komponente das oder die Antigen(e) und gegebenenfalls spezifische, vorzugsweise monoklonale Antikörper auf.

Die erfindungsgemäßen Testkits zum Nachweis von Antikörpern gegen Borrelia burgdorferi sind dadurch gekennzeichnet, daß sie wenigstens ein oben definiertes, erfindungsgemäßes immunologisch aktives Protein enthalten, das ohne Verunreinigung durch andere Proteine aus dem Borrelia burgdorferi Stamm zur Verfügung steht. Dieses immunologisch aktive Protein wirkt als Antigen und reagiert mit den in der Untersuchungsflüssigkeit vorhandenen Antikörpern. Vorzugsweise weisen die erfindungsgemäßen Testkits zwei bis vier immunologisch aktive Proteine auf, die ohne Verunreinigung durch andere Proteine aus B. burgdorferi zur Verfügung stehen. Weiterhin enthält das Testkit eine Anzeigekomponente, die das Vorhandensein von Komplexen aus Antigen und Antikörper ermöglicht.

Die erfindungsgemäßen Testkits können auf verschiedenen, an sich bekannten Prinzipien beruhen. Grundsätzlich kann das Antigen eine Markierung tragen, wobei die Markierung aus einem radioaktiven Isotop oder einem Enzym bestehen kann, das eine Farbreaktion katalysiert. Ebenso kann das Antigen an eine feste Unterlage (Mikrotiterplatten oder Kügelchen) gebunden sein und die Anzeigekomponente kann in einem gegen Antikörper gerichteten Antikörper bestehen, der eine Markierung trägt, wobei die Markierung in einem radioaktiven Isotop oder einem Enzym bestehen kann, das eine Farbreaktion katalysiert.

Im Rahmen der vorliegenden Erfindung wird als Testkit der sogenannte ELISA (Enzyme-linked immunosorbent assay) bevorzugt. Eine Ausführungsform davon wird in Beispiel 5 näher beschrieben. Die Ergebnisse dieses Beispiels zeigen, daß überraschenderweise durch den Einsatz nur eines erfindungsgemäßen immunologisch aktiven Proteins eine sehr hohe Spezifität des Testkits erreicht werden konnte. Darüber hinaus ermöglichen die erfindungsgemäßen Testkits überraschenderweise eine mit dem Krankheitsstadium korrelierte Differenzierung. Der kombinierte Einsatz von mehreren Antigenen in einem Testkit ermöglicht den Nachweis von Antikörpern gegen Borrelia burgdorferi auch in solchen Fällen, in denen sich die Krankheitssymptome noch nicht klinisch manifestiert haben. Ebenso können Infektionen mit B. burgdorferi diagnostiziert werden, bei denen der Patient nur eine subklinische Infektion durchläuft. Die Aussage, die durch die erfindungsgemäßen Testkits erhalten werden kann, ist insbesondere in den Fällen bedeutsam, in denen ein Zeckenbiß festgestellt werden konnte, jedoch nicht klar ist, ob eine Infektion mit einem Borrelien-Stamm vorliegt.

Im Rahmen der vorliegenden Erfindung ist der kombinierte Einsatz von mehreren der immunologisch aktiven Proteine bevorzugt. Ganz besonders bevorzugt ist eine Kombination der Proteine, pC, und p100. Durch die Verwendung der erfindungsgemäß bevorzugten ELISA-Testkits kann auch eine Differenzierung hinsichtlich der Natur der Antikörper erfolgen. Sollen beispielsweise IgM-Antikörper nachgewiesen werden kann der sogenannte µ-capture-assay angewendet werden, dabei werden gegen IgM-Antikörper gerichtete Antikörper an die feste Phase gebunden. Nach Inkubation der Testplatten mit der zu untersuchenden Flüssigkeit werden die in der Untersuchungsflüssigkert vorhandenen IgM-Antikörper an die feste Phase gebunden. Nach Absättigung unspezifischer Bindungen kann dann ein immunologisch aktives Protein der vorliegenden Erfindung zugegeben werden. Dieses Antigen wird dann durch ein Anzeigemolekül nachgewiesen. Hierbei kann das Antigen biotinyliert sein, wobei anschließend Avidin zugegeben wird, das kovalent gebundene Peroxydase aufweist. Die Peroxydase katalysiert dann eine Reaktion, die zur Farbbildung führt.

Eine weitere Möglichkeit besteht darin, daß solche monoklonalen Antikörper zu dem Komplex Träger/anti-IgM-Antikörper/nachzuweisender Antikörper/erfindungsgemäßes Antigen gegeben werden, die spezifisch für das Antigen sind und biotinyliert sind. Die Biotinylierung ist beispielsweise in Monoklonale Antikörper (1985) Springer Verlag, J.H. Peters et al. beschrieben. Der Nachweis des Komplexes erfolgt darin durch Zugabe von Avidin, an das ein eine Farbreaktion katalysierendes Enzym gekoppelt ist.

Eine andere Ausführungsform der vorliegenden Erfindung besteht darin, daß der IgM-Nachweis durch indirekten ELISA geführt wird. Dabei werden die erfindungsgemäßen Antigene auf Mikrotiterplatten gebunden, mit der zu untersuchenden Flüssigkeit inkubiert und nacn Waschen erfolgt der Nachweis der Immunkomplexe mittels anti-µ-Konjugat.

Es können auch monoklonale Antikörper hergestellt werden, die gegen die immunologisch aktiven Proteine von Borrelia burgdorferi gerichtet sind. Die Herstellung derartiger monoklonaler Antikörper ist in Beispiel 6 näher erläutert. Verwendet werden können derartige monoklonale Antikörper als Reagenzien für den direkten Erregernachweis. Es können aber auch monoklonale Antikörper an die feste Phase einer Mikrotiterplatte gekoppelt werden. Nach Zugabe der immunologisch aktiven Proteine (Antigene) werden diese durch Antikörper-Antigen-Bindung an die Mikrotiterplatte fixiert. Anschließend wird die Untersuchungsflüssigkeit (bei der es sich beispielsweise um Serum oder Liquor handeln kann) zugegeben. Die in der Untersuchungsflüssigkeit vorhandenen Antikörper binden sich dann an das Antigen und lassen sich mit Hilfe einer Anzeigekomponente nachweisen.

Darüber hinaus lassen sich die monoklonalen Antikörper sehr gut zur Reinigung der immunologisch aktiven Proteine (Antigene) verwenden. Vorteilhaft hierbei ist, daß die Reinigung besonders schonend ist. Hierzu werden die monoklonalen Antikörper an eine feste Matrix gebunden. Vorzugsweise liegt diese feste Matrix in Form einer Säule vor. Anschließend werden die partiell vorgereinigten Antigene bei physiologischen Bedingungen mit den an eine feste Matrix gekoppelten Antikörpern versetzte. Nach Waschen des Matrix-Antikörper-Antigen-Komplexes können die Antigene eluiert werden. Üblicherweise werden hierzu hohe Salzkonzentrationen oder Puffer mit einem solchen pH-Wert verwendet, der die Elution ermöglicht.

Es können auch DNA-Sequenzen zur Verfügung gestellt, die der Aminosäuresequenz der immunologisch aktiven Proteine ganz oder zum Teil entsprechen. Diese DNA-Sequenzen können bevorzugt zum Nachweis von Borrelien-Stämmen im Untersuchungsmaterial durch Hybridisierung verwendet werden. Dazu wird ein Oligonukleotid hergestellt, das der DNA-Sequenz zum Teil entspricht. Dieses Oligonukleotid wird radioaktiv markiert. Andererseits wird die DNA aus dem Untersuchungsmaterial an einen geeigneten Filter, vorzugsweise Nitrocellulose gebunden und anschließend mit dem radioaktiv markierten Oligonukleotid hybridisiert. Ebenso können die erfindungsgemäßen DNA-Sequenzen für die in-situ Hybridisierung zum direkten Nachweis von B. burgdorferi in infiziertem Gewebe verwendet werden. Anstelle der chemisch synthetisierten Oligonukleotide können auch entsprechende DNA-Fragmente in Bakterien vermehrt werden und anschließend aus den Vektoren mit Hilfe von Restriktionsendonukleasen ausgeschnitten werden. Nach Isolierung dieser DNA-Fragmente können diese radioaktiv markiert werden und wie oben beschrieben zur Hybridisierung verwendet werden.

Ein weiterer Aspekt der vorliegenden Erfindung besteht darin, daß die erfindungsgemäßen immunologisch aktiven Proteine (Antigene) von Borrelia burgdorferi als Impfstoffe verwendet werden können. Dazu werden die erfindungsgemäßen Antigene in reiner Form dargestellt. Anschließend werden sie einzeln oder in Kombination mit oder ohne ein die Immun-Antwort-stimmulierendes Agens der zu impfenden Person appliziert. Hierdurch wird die Bildung von Antikörpern angeregt, die spezifisch gegen Borrelia burgdorferi-Stämme sind.

Die erfindungsgemäßen Proteine können in verschiedenen Bereichen Verwendung finden. So können die erfindungsgemäßen Testkits auch zum Nachweis von B. burgdorferi-Infektionen bei Tieren verwendet werden und die Proteine Können auch zur Impfung von Tieren, insbesondere von wertvollen Tieren, verwendet werden.

Soweit die vorliegende Erfindung Proteine von Borrelia burgdorferi betrifft kann es sich auch um Proteinfragmente handeln, die lediglich eine Teilsequenz der vollständigen Aminosäuresequenz aufweisen. Derartige Teilsequenzen weisen wenigstens 10 Aminosäuren und bevorzugt wenigstens 15 Aminosäuren auf.

Üblicherweise sind jedoch die Proteinfragmente größer.

Im Rahmen der vorliegenden Erfindung werden die erfindungsgemäßen Proteine mit einem Molekulargewicht von etwa 22 kD bzw. 100 kD besonders bevorzugt. Diese Proteine können auch von anderen Borrelia burgdorferi Stämmen abstammen, sofern sie der o.g. Definition entsprechen.

Anhand der folgenden Tabellen, Abbildungen und Beispiele werden die bevorzugten Ausführungsformen der vorliegenden Erfindung näher erläutert.

Das in den Beispielen erwähnte Protein p41 ist nicht Gegenstand der Erfindung.

### Beispiel 1:

### Bestimmung der immunrelevanten und Genus-spezifischen Borrelienproteine

Es wurde nach spezifischen, häufig auftretenden Serum-Antikörpern gesucht, die gegen bestimmte einzelne B. burgdorferi Antigene gerichtet sind, möglichst wenig Kreuzreaktivität mit Proteinen verwandter Erreger zeigen und zusätzlich auch eine Korrelation mit den einzelnen Lyme-Borreliose-Krankheitsstadien zulassen.

Die Suche nach häufig erkannten Antigenen erfolgte mittels Westem Blot. Dazu wurde ein Bakterienextrakt von B. burgdorferi (Stamm PKo) (Preac-Mursic, V.; Wilske, B.; Schierz, G. (1986): European Borreliae burgdorferi isolated from humans and ticks - culture conditions and antibiotic susceptibility. Zbl. Bakt. Hyg. A 163, 112-118) nach Pelletieren, Resuspendieren in PBS/NaCl und Ultraschallbehandeln im SDS-Polyacrylamidgel elektrophoretisch aufgetrennt (Laemmli, U.K. (1970): Cleavage of structural proteins during the assembly of the head of bacteriophage T4. Nature 227,680-685)

Die Gele bestanden aus einem Sammelgel mit Taschen für die Proben und einem Trenngel. Die Zusammensetzung der Trenngele war folgendermaßen: 15 % Acrylamid (Bio-Rad), 0.026 % Diallyltartardiamid (DATD, Bio-Rad) pro Prozent Acrylamid, 0.15 % SDS. 375 mM Tris-HCl pH 8.5, 0.14 mM Ammoniumperoxodisulphat (AMPER, Bio-Rad) und 0.035 % N,N,N', N'-Tetramethylethylendiamin (TEMED, Bio-Rad). AMPER und TEMED dienten dabei als Radikalstarter für die Polymerisation. 2-4 h nach Polymerisation wurde das Sammelgel (3.1 % Acrylamid, 0.08 % Diallyftartardiamid, 0.1 % SDS, 125 mM Tris-HCl pH 7.0, 3 mM AMPER und 0.05 % TEMED) über das Trenngel gegossen und mit einem Teflonkamm versehen. Die Anoden- und Kathodenkammer wurde mit identischer Pufferlösung gefüllt: 25 mM Tris-Base, 192 mM Glycin und 0.1 % SDS, pH 8.5.

Es wurden jeweils 20 µl Probe in Lysispuffer (3 % Saccharose, 2 % SDS, 5 % β-Mercaptoethanol, 20 mM Tris-HCl pH 7.0, Bromphenolblau: 5 min. bei 100°C erhitzt) pro Tasche aufgetragen. Die Elektrophorese wurde bei Raumtemperatur über Nacht mit konstanter Stromstärke von 6mA für Gele der Größe 20x15cm durchgeführt. Anschießend wurden die Gele auf Nitrocellulose (NC) transferriert.

Für den Proteintransfer befand sich das Gel mit der anliegenden Nitrocellulose zwischen Whatman 3MM Filterpapier, leitfähigem, 1 cm dicken Schaumstoff und zwei Kohleplatten, die über Platinelektroden den Strom leiteten. Filterpapier, Schaumstoff und Nitrocellulose wurden gut mit Blot-Puffer (192 mM Glycin, 25 mM Tris-Base, 20 % Methanol. pH 8.5) getränkt. Der Transfer fand bei 2 mA/cm² für 2 h statt. Freie Bindungsstellen auf der Nitrocellulose wurden für 1 h bei 37°C mit Cohen-Puffer (1 mg/ml Ficoll 400, 1 mg/ml Polyvinylpyrrolidon, 16 mg/ml Rinderserumalbumin, 0.1% NP 40, 0.05% Bacto-Gelatine in Natriumboratpuffer pH 8.2); (Cohen G.H., Dietzschold, B., Ponce de Leon, M., Long, D., Golub. E.,Varrichio, A., Pereira, L. and Eisenberg, R.J.:Localisation and synthesis of an antigenic determinant of Herpes simplex virus glycoprotein D that stimulates the production of neutralizing antibodies. J. Virol. 49 (1984) 4183-4187) abgesättigt. Der Blot wurde über Nacht bei Raumtemperatur mit den Patientenseren (Verdünnung 1:100 in 154 mM NaCl und 10 mM Tris-HCl pH 7.5) unter Schütteln inkubiert.

Nach der Seruminkubation wurde der Blot unter mit TTBS (50 mM Tris-HCl pH7.5, 500 mM NaCl, 0,01 % Tween 20) viermal für je 15 min gewaschen. Anschließend wurde der Blot mit Peroxidasegekoppeltem anti-human-Immunglobulin (DAKO, Hamburg, Verdünnung 1:1000 in 154mM NaCl und 10mM Tris-HCl, pH7.5) für 2 h bei Raumtemperatur inkubiert. Nach mehrmaligem Waschen mit TTBS wurde der Blot mit 0.5 mg/ml Diaminobenzidin und 0.01 % Wasserstoffperoxid in 50 mM Tris-HCl pH 7.5 gefärbt. Die Färbung wurde anschließend mit 1 N Schwefelsäure gestoppt, der Blot mit Wasser säurefrei gewaschen und zwischen Filterpapier getrocknet.

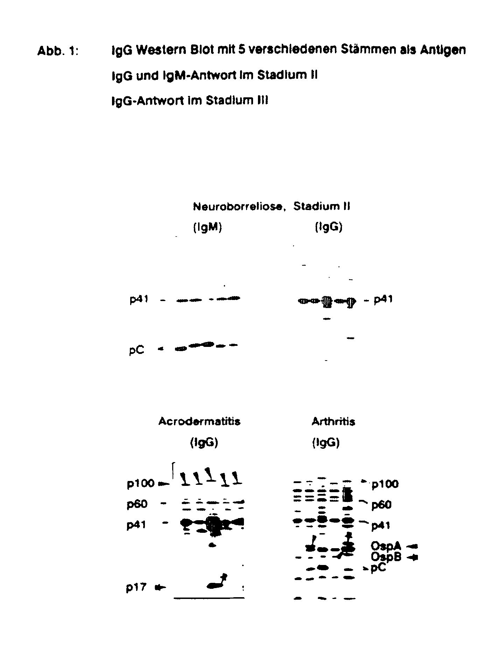

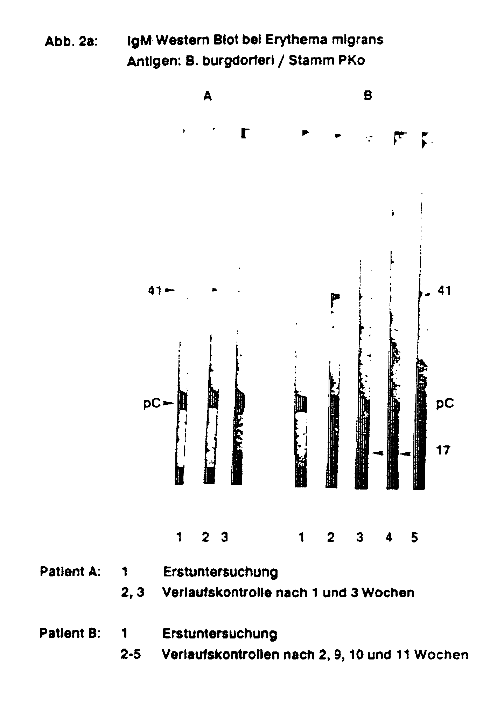

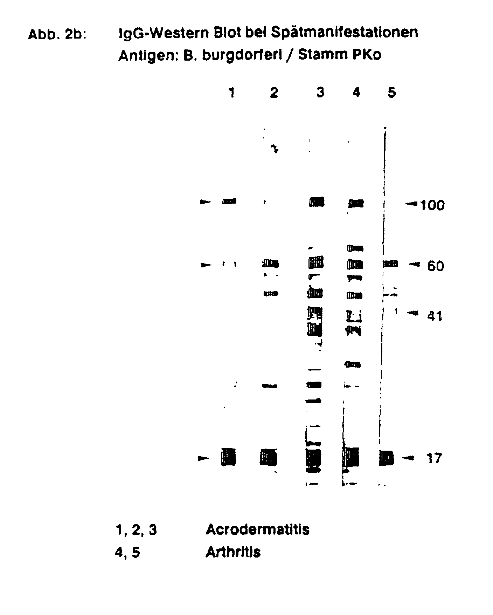

Eine Auswahl der Reaktionsmuster verschiedener Seren mit den Westem Blot Streifen ist in den Abbildungen 1, 2a und b gezeigt.

Als immundominant erwiesen sich dabei folgende Proteine: p17 (17kDa), pC (22kDa) p41 (41 kDa) und p100 (100kDa mit Größenvariation in verschiedenen B.burgdorferi-Isolaten). Bis auf p41 sind die biologischen Funktionen dieser Antigene unbekannt: p41 stellt das Flagellin Protein dar (Barbour, A.G.S., Hayes, S.F, Heiland, R.A., Schrumpf, M.E. and Tessier, S.L.: A Borrelia genus-specific monoclonal antibody binds to a flagellar epitope. Infect. Immun. 52 (1986) 549-554.).

Auf Grund dieser Analysen, die mit einer größeren Anzahl von Patientenseren aus den verschiedenen Krankheitsstadien durchgeführt wurde, ergaben sich Anhaltspunkte, daß mit einem einzigen Antigen nicht immer alle mit B. burgdorferi Infizierten erfaßt werden. Es zeigte sich, daß vor allem bei Seren mit IgM-Antikörpern (frische Infektion) neben dem Flagellin (p41) noch ein Protein (pC) im 22 kD Bereich besonders häufig erkannt wird. Das gleichzeitige Auftreten beider Antikörper war jedoch nicht zwingend. Es konnten sowohl Seren, die nur Antikörper gegen p41 oder nur Antikörper gegen das pC-Protein besaßen, gefunden werden (Abb. 1 und 2a, Western Blots). Bei der Neuroborreliose ist der Nachweis der intrathekal gebildeten Antikörper im Liquor von großer Bedeutung. IgG-Western Blots bei 12 Liquor-/Serum Paaren von Patienten mit einer lymphozytären Meningoradikulitis Bannwarth zeigten in allen Fällen eine lokale intrathekale Immunantwort gegen p41. Im Spätstadium wurden neben IgG-Antikörpern gegen das Flagellin vor allem Antikörper gegen Proteine im 100 kD Bereich (p100) und im 17 kD Bereich (p17) gefunden, die in den Frühstadien nicht oder nur selten nachweisbar waren. Somit sind Antikörperreaktivitäten gegen die p17- und p100-Proteine gute Marker für das Erreichen des Stadiums III (Abb. 2b, Westem Blot).

Mit Hilfe dieser vier Antigene kann eine bessere Standardisierung der Teste erreicht werden.

Die Proteine p41, pC und p17 zeigen zudem nur eine geringe Kreuzreaktivität zu anderen Bakterienstämmen, das Protein p100 erwies sich als Genus-spezifisches Protein mit B. burgdorferi spezifischen Epitopen. Zusammenfassend sind in Tab. 2 (Reaktivität von Immunseren gegen verschiedene bakterielle Erreger mit Proteinen von B.burgdorferi) die Kreuzeaktivität von Seren gegen verschiedene verwandte Erreger mit B.burgdorferi-Antigenen nach Westem Blot Analyse aufgeführt. Bei Versuchen, die vier Proteine (p41, pC, p17, p100) aus B. burgdorferi Extrakten zu reinigen, zeigte sich, daß große Mengen an Ausgangsmaterial benötigt werden. Besondere Schwierigkeit bereitete die Reinigung von p100, das im Gesamtextrakt unterrepräsentiert ist. Da die Kultivierung aufwendig und teuer ist, mußte nach gentechnologischen Möglichkeiten zur Herstellung dieser Antigene gesucht werden. Die Analyse von Patientenseren mittels Westem Blot hat gezeigt, daß mit einer Kombination von gentechnologisch produzierten p41, pC, p17 sowie p100 als Antigen eine nahezu vollständige Erfassung aller positiven Seren erfolgen kann, und weiterhin eine Korrelation zum Krankheitsstadium gegeben ist.

### Beispiel 2:

### Gentechnologische Produktion von p41 (Flagellin) aus B. burgdorferi in Escherichia coli

Der kodierende Bereich von p41 wurde mittels DNA-Amplifizierung durch eine "polymerase chain reaction" (PCR) aus einer B. burgdorferi (DSM-Nr. 5662 P/Ko2/85) Gesamt-DNA Präparation gewonnen. Die so erhaltene Sequenz wurde anschließend unter die Kontrolle von induzierbaren Promotoren gestellt und nach Transfektion in E.coli zur Expression gebracht (Maniatis, T.; Fritsch, E.F.; Sambrook, J. (1982) Molecular cloning. Cold Spring Harbor).

Hierfür wurden die B. burgdorferi Zellen 2 Wochen bei 37°C in 2 l modifiziertem Barbour-Stoenner-Kelly-(BSK) Medium (Preac-Mursic. V: Wilske, B.; Schierz, G. (1986): European Borreliae burgdorferi isolated from humans and ticks - culture conditions and antibiotic susceptibility. Zbl. Bakt. Hyg. A 163, 112-118) kultiviert, bei 6000 rpm pelletiert, in TEN-Puffer (10 mM Tris-HCl pH 7,6; 1 mM EDTA: 10 mM NaCl) gewaschen und in 20 ml Lysozym Puffer (20 % Saccharose, 50 mM Tris-HCl pH 7,6, 50 mM EDTA, 5 mg/ml Lysozym) resuspendiert. Nach Inkubation für 30 Min. bei 37'C wurden die durch die Einwirkung des Lysozyms auf die Zellwand entstehenden Protoplasten durch Zugabe von 1 ml 25 % SDS (Natriumdodecylsulfat) lysiert. Nach weiteren 10 min. wurden 4 ml einer 5 M NaCl Lösung hinzugegeben. Protein wurde durch Zugabe von einem gleichen Volumen TE-gesättigten (TE: 10 mM Tris/HCl, pH7, 8,1 mM EDTA) Phenol denaturiert. Durch Zentrifugation bei 4°C und 6500 rpm für 5 min. erfolgte die Separation der Phasen. Die obere DNA-haltige, wässrige Phase wurde mit einer Pipette mit weiter Öffnung (zur Vermeidung von Scherkräften) vorsichtig in ein frisches Röhrchen überführt und anschließend nochmals mit demselben Volumen an Phenol/Chloroform/Isoamylalkohol (1:1:0,04) extrahiert. Nach der Separation wurde wiederum die obere wässrige Phase vorsichtig in ein neues Röhrchen überführt und die DNA mit dem zweifachen Volumen Ethanol präzipitiert. Nach ca. 5 min. wird die als lange, fädige Gebilde ausgefallene DNA durch Aufwickeln an einem Glasstab entfernt und in eine 70 %ige Ethanollösung zum Waschen überführt. Die an dem Glasstab durch Adhäsion gebundene DNA wurde anschließend 2 h bei Raumtemperatur gelagert, um ein Abdampfen des Ethanols zu bewirken, und dann in 4 ml TEN-Puffer überführt

Je 1 µl der so erhaltenen B. burgdorferi Gesamt-DNA wurde in fünf 100µl PCR Ansätzen amplifiziert.

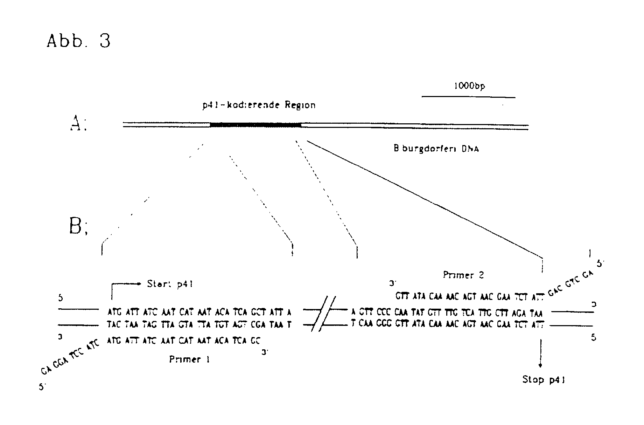

Als spezifische Primer für die Polymerase katalysierte Amplifikation wurden Sequenzen gewählt. die die Information für den translationalen Start sowie das 3'-Ende von p41 (Flagellin) enthielten. Es wurden hierfür die in Abb. 3 gezeigten DNA-Sequenzen verwendet. Die beiden Oligodesoxynukleotide wurden auf einem Milligen/Biosearch DNA-Synthesizer 8700 in 1µmol Säulen synthetisiert, nach der Abspaltung mit Ammoniak durch Ethanol-Fällung grob gereinigt und in je 400 µl H₂O aufgenommen. Je 1 µl dieser Oligodesoxynukleotid-Lösung wurden pro PCR-Ansatz eingesetzt; die Puffer, Nukleotide und die Taq-Polymerase stammten aus einem kommerziell erhältlichen Testkit (Cetus/Perkin-Elmer, Überlingen) und wurden auch nach den Testbeschreibungen verwendet. Die Temperaturbedingungen für die einzelnen Zyklen waren:
2 min. Denaturierung bei 94°C
2 min. Annealing bei 45°C
4 min. DNA-Synthese bei 73°C
50 Zyklen wurden durchgeführt.

Die Ansätze aus den PCRs wurden anschließend vereinigt, und die DNA nach Zugabe von NaCl in einer Endkonzentration von 0,2 M mit dem 2,5 fachem an Ethanol bei -20°C für 5 h gefällt. Nach der Pelletierung und Waschen in 70 % Ethanol wurde die DNA in 200µl H₂O gelöst und nach Zugabe entsprechender Puffer mit den Restriktionsenzymen Bam Hl und Pst I (Boehringer Mannheim) nach den Angaben des Herstellers gespalten.

Nach der gefelektrophoretischen Auftrennung in einem 1,5% Agarosegel wurde das amplifizierte DNA Fragment (ca. 1000 bp) isoliert und in einen mit BamHl und Pstl geschnittenen pUC8 Vektor (Pharmacia) (Vieira, J.; Messing, J. (1982): The pUC plasmids, and M13mp7-derived system for insertion mutagenesis and sequencing with synthetic universal primers. Gene 19, 259-268) inseriert, wobei 0.25µg des Vektors, 0,5µg des p41 Fragments und 2U T4-DNA-Ligase mit Puffer nach Angabe des Herstellers (Boehringer Mannhein) eingesetzt wurden.

Nach Transformation der ligierten DNA Fragmente in den E. coli Stamm JM 109 (Phamacia) (Yanisch-Perron, C.; Vieira, J.: Messing, J. (1985): Improved M13 phage cloning vectors and host strains: nucleotide sequences of the M13mp18 and pUC19 vectors. Gene 33, 103-119) und Ausplattieren auf Agar-Platten mit Ampicillin (50 µg/ml) und X-Gal (30 µg/ml) wurden weiße Kolonien in 5 ml L-Broth Medium hochgezogen, und die isolierten Plasmide mittels Restriktionsenzym-Spaltung auf deren inserts untersucht.

Das B.burgdorferi Flagellin-kodierende DNA-Fragment sitzt damit hinter dem induzierbaren lacUV5 Promoter des Vektors im selben Leserahmen wie das von diesem Promoter gestartete lacZα-kodierende Transkript. Dadurch entsteht ein Flagellin, welches an seinem N-Terminus einige pUC8-kodierte Aminosäuren enthält. Dieser Bereich ist nachfolgend aufgeführt:

ATG ACC ATG ATT ACG AAT TCC CGG GGA TCC ATC ATG ATT
MET THR MET ILE THR ASN SER ARG GLY SER ILE MET ILE
pUC8 p41

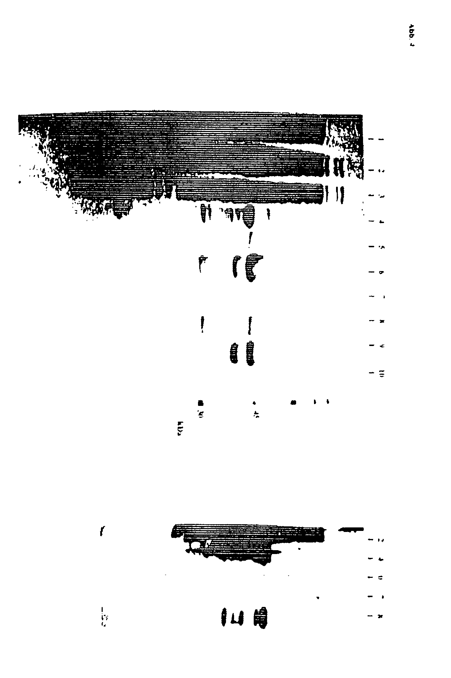

Von positiven E.coli Klonen (z. B. pUC8ly13) die Vektor mit DNA Insert in der erwarteten Länge (1000 bp) enthielten wurden wiederum Flüssigkulturen angelegt, und die Transkription von dem lac-Promotor des Plasmids durch 3-stündige Induktion mit 1 mM IPTG bei 37°C und Schütteln induziert. 1.5 ml dieser Kulturen wurden dann kurz pelletiert, die Baktenen mit "boiling mix" (3 % Saccharose, 2 % SDS, 5 % β-Mercaptoethanol, 20 mM Tris-HCl pH 7,0, 2% Bromphenolblau) bei 100°C für 10 min. lysiert, und die Proteine mittels 17,5% SDS-PAGE aufgetrennt. Nach Anfärben der Proteine durch Coomassie brilliant Blau zeigte sich bei den Zellen mit Plasmid-Insert eine neue. zusätzliche Bande bei ca. 41 kD, die der erwarteten Größe des Flagellins entspricht. Eine spezifische Reaktion dieses rekombinanten Antigens mit einem Serum eines Lyme Borreliose Patienten sowie mit einem monoklonalen Antikörper gegen B. burgdorferi p41 Flagellin ist in dem in Abb.4 gezeigten Immunoblot nachgewiesen.

Ebenso wie pUC8 ist auch jedes andere induzierbare Plasmid, das ein Transkript in demselben Leserahmen startet, für die Produktion von p41 geeignet. Durch Spaltung des p41-kodierenden Bereichs am Translationsstart mit BspHl (TC ATG A) und Pstl (am 3'-Ende) und Einsetzen des Fragments in die Ncol-Stelle (CC ATG G) und Pstl-Stelle eines sogenannten ATG-Vektors ist auch die Expression eines authentischen p41 möglich, welches keinerlei Fremdaminosäuren anfusioniert hat.

Für die nachfolgend aufgezeigten Verfahren wurde der Klon pUC8ly17 verwendet.

### Beispiel 3:

### Produktion von pC, OspA und p100 in E. coli aus B. burgdorferi Genbanken

Zur Bereitstellung B. burgdorferi spezifischer DNA-Sequenzen wurde in E. coli eine chromosomale Gen-Bank angelegt. Mit Hilfe geeigneter Methoden wie Immunoscreening oder Hybridisierung mit ausgewählten Oligonukleotiden konnten in dieser Gen-Bank E. coli Klone identifiziert werden, welche entsprechende B. burgdorferi spezifische DNA-Sequenzen enthielten. Nach Restriktions-Enzym-Analyse wurde eine Restriktions-Enzym Karte erstellt. Diese konnte verwendet werden, um die gesuchten DNA-Sequenzen gezielt in ExpressionsVektoren zu überführen bzw deren Sequenzierung durchzuführen. Dabei wurde im Einzelnen wie folgt vorgegangen: Zur Isolierung von B. burgdorferi (DSM-Nr. 5662) DNA (chromosomale DNA wie Plasmid DNA) wurden die Zellen wie unter Beispiel 2 beschrieben kultiviert. Nach Zentrifugation bei 12000 Upm für 20 Minuten wurden die Zellen gewaschen und in SET Puffer (20 % Saccharose, 50 mM Tris-HCl pH 7,6; 50 mM EDTA) resuspendiert. Durch Zugabe von 15 mg/5 ml Lysozym für 20 Minuten wurde die Zellwand partiell gespalten. Anschließend wurden die protoplastierten Zellen durch Zugabe von SDS (n-Dodecylsulfat Natriumsalz) Endkonzentration 1 % lysiert. Nach 20 Minuten bei 37°C wurde Proteinase K (Endkonzentration 1 mg/ml) für zweimal 1 Stunde zugesetzt und die DNA enthaltende Losung auf 100 mM NaCl mit TEN-Puffer (10 mM Tris-HCl pH 7,6, 1 mM EDTA, 300 mM NaCl) eingestellt. Es erfolgten eine Phenol-Extraktion und zwei weitere Phenol/Chloroform/iso-Amylalkohol-Extraktionen (Phenol:Chloroform im Verhältnis 1:1; chloroform:iso-Amylalkohol im Verhältnis 24:1). Der so extrahierte Überstand wurde mit 2,5 vol. 95% Ethanol versetzt und bei -20°C die DNA präzipitiert. Durch Aufwickeln auf einen Glasstab konnte die fädig ausgefallene DNA gewonnen und in 70 % Ethanol gewaschen werden. Nach kurzer Trocknung im Exsiccator wurde die DNA in TE-Puffer (10 mM Tris-HCl pH 7,6, 1 mM EDTA) der RNAse (20 µg/ml) enthielt aufgenommen. Die so präparierte DNA wurde für die weiteren Schritte verwendet.

B. burgdorferi DNA wurde mit dem Restriktionsenzym Sau 3A (Boehringer Mannheim) nach den Angaben des Herstellers inkubiert. Durch die Wahl geeigneter Enzym-Verdünnungen bzw. Einwirkzeit des Enzyms auf DNA wurde eine partielle Spaltung derselben erreicht. So erhaltene partiell gespaltene DNA wurde mit, BamH I-restringierter und durch Behandlung mit alkalischer Phosphatase dephosphorylierter Vektor DNA (pUC18 oder andere geeignete Vektor DNA) ligiert. Dazu wurde T4 DNA-Ligase (Boehringer Mannheim) nach Angaben des Herstellers eingesetzt. Pro Transformations-Ansatz wurden 0,2 - 0,5 µg/µl Gesamt DNA eingesetzt. E. coli JM 109 (oder andere geeignete E. coli Stämme) wurden nach dem Protokoll von Hanahan (Hanahan, D. (1985): Techniques of Transformation of Escherichia coli, S. 109-135. In: D.M. Glover (Hrsg.) DNA cloning, Vol.1. A practical approach. IRL Press, Oxford) bzw. nach Maniatis et.al. (Maniatis, T. (1982): Molecular cloning: a laboratory manual. Cold Spring Harbor Laboratory, Cold Spring Harbor, New York) mit der ligierten DNA transformiert. Rekombinante E.coli Klone wurden auf LB-Medium (10 g Trypton, Difco, 5 g Hefeextrakt, Difco, 5 g NaCl, Merck), welches 100 µg/ml Ampicillin (oder ein anderes geeignetes Antibiotikum) enthielt, selektioniert und kultiviert. Das Koloniemuster wurde identisch auf LB-Platten übertragen und Kolonieabdrücke auf Nitrocellulose erstellt. Die Zellen dieser Kolonieabdrücke wurden je nach angewandtem Screening Verfahren auf dem Filter unterschiedlich lysiert. Bei Verwendung von mono- bzw. polyklonalen Seren (Immunoscreening) zur Detektion B. burgdorferi spezifischer Genprodukte, die durch die einrekombinierte DNA, induziert werden, wurden die Zellen 15 min über gesättigtem Chloroform-Dampf lysiert. Nach Absättigung des so behandelten Filters mit einer Magermilch-Lösung für 2 Stunden wurden die Filter über Nacht mit den verschiedenen Seren inkubiert, mehrmals mit TTBS-Puffer gewaschen (s.o.) und für 2 Stunden mit dem zweiten Peroxidase konjugierten Antikörper (Dako, Hamburg) inkubiert. Erneutes Waschen mit TTBS-Puffer diente zur Reduzierung unspezifisch gebundener Peroxidase konjugierter Antikörper. Durch enzymatische Umsetzung der Substrate Diaminobenzidin (Sigma-Chemie, München) und H₂O₂ in ein unlösliches braunes Pigment konnten positive, d.h. B. burgdorferi Antigen produzierende E. coli Klone erkannt werden. Die so erkannten positiven E.coli Klone wurden von der Ausgangs-Platte angeimpft und analysiert. Bei Verwendung von spezifischen Oligonukleotiden zur Hybridisierung und damit zur Detektion spezifischer B. burgdorferi Antigen Sequenzen (Screening durch Hybridisierung) wurden die Zellen auf dem Nitrocellulose Filter (Schleicher & Schuell) alkalisch lysiert (durch Benetzen der Filter für 5 Minuten mit 0,5 M NaOH, 1,5 M NaCl). Nach Neutralisierung (durch Benetzen der Filter für 5 Minuten in 1.5 M NaCl 0,5 M Tris-HCl pH 8,0) wurden die Filter mit der denaturierten DNA mit 2x SSPE (20x SSPE: 3.6 M NaCl. 200 mM NaH₂PO₄, pH 7,4, 20 mM EDTA, pH 7,4) benetzt und getrocknet. Durch Backen der Filter für 2 Stunden bei 80°C wurde die DNA fixiert. Die so behandelten Filter wurden dann für die Hybridisierung eingesetzt. Die Hybridisierung erfolgte unter Verwendung radioaktiver (³²P) bzw. nicht radioaktiver (z.B. Digoxigenin, Boehringer Mannheim) Nachweismethoden. Hierbei wurde nach bekannten (Maniatis, T (1982): Molecular cloning: a laboratory manual. Cold Spring Harbor Laboratory, Cold Spring Harbor) bzw, vom Hersteller (Boehringer Mannheim) empfohlenen Markierungsmethoden (³²P-Markierung mit ³²P-gamma-ATP und Kinasereaktion bzw Digoxygenin-Markierung mit Dig-11-UTP und terminaler Transferase-Reaktion) verfahren. Von positiven E. coli Klonen wurde eine Restriktions-Enzym-Analyse erstellt und mit dieser Information eine Expression der Antigen kodierenden DNA-Sequenzen in geeigneten Vektoren bzw. deren Sequenzierung durchgeführt.

Als Hybridisierungsproben wurden zu Beginn synthetische Oligodesoxynukleotide eingesetzt, deren Sequenz anhand von p100 und pC Aminosäuren-Sequenzen ausgewählt wurden. Dabei wurde folgendermaßen vorgegangen:

Aus Lysaten von B. burgdorferi wurden die beiden Proteine durch Extraktion mit n-Octyl β-D-Thioglucopyranosid partiell aufgereinigt und weiter durch SDS-Polyacrylamid-Gel-Elektrophorese aufgetrennt. Anschließend wurden die Antigene durch Western Blotting auf eine Glasfibermatrix übertragen und die entsprechenden Stücke mit den B. burgdorfen-Proteinen ausgeschnitten. p100 wurde dann N-terminal ansequenziert und die ersten 22 Aminosäuren des Aminoterminus bestimmt (Diese Methode des "micro-sequencing" ist beschrieben in: Eckerskorn, C., Mewes, W., Goretzki, H. and Lottspeich, F.: A new siliconized fiber as support for protein-chemical analysis of electroblotted proteins. Eur. J. Biochem. 176 (1988) 509-519). Bei pC war eine direkte Ansequenzierung nicht möglich, da der N-Terminus einer Sequenzierung nicht direkt zugänglich ist, d.h. daß hier eventuell eine Myristilierung oder ähnliche Modifikationen vorliegen. Deshalb wurde dieses Protein tryptisch gespalten, die Fragmente mittels HPLC aufgetrennt und zwei davon dann ansequenziert. Aus den so gewonnenen Aminosäure-Abfolgen wurden dann die nachfolgend aufgeführten Oligodesoxynukleotid-Sequenzen abgeleitet. Da meist mehrere Codon-Möglichkeiten für eine Aminosäure bestehen, mußten an den entsprechenden Stellen des Oligonukleotids auch die Basenvariationen berücksichtigt werden und während der Synthese in äquimolaren Verhältnissen eingebaut werden.
p100-p1 - p100 - Aminosäuren-Sequenz: 
Glu Leu Asp Lys Glu Lys Leu Lys Asp Phe Val Asn Leu Asp Leu
Glu Phe Val Asn Thr
 p100 - Oligodesoxynukleotid-Sequenz, die in Klammern angegebenen und durch '';" getrennten Basen wurden während der Synthese (auf Milligen/Biosearch 8700 DNA Synthesizer) in äquimolaren Verhältnissen eingebaut:

GA(G;A)  (C;T)T(G;T;A)  GA(C;T)  AA(G;A)  GA(G;A)  AA(G;A)
(C;T)T(G;T;A)  AA(G;A)  GA(C;T)  TT(C;T)  GT(T;A)  AA(C;T)
(C;T)T(G;T;A) GA(C;T) (C;T)A(G;T;A) GA(G;A) TT(C;T) GT(T;A)
AA(C;T) TA(C;T) A

Die Oligodesoxynukleotid-Sequenz wurde als Sonde verwendet und mit den die B. burgdorferi DNA enthaltenden Klonen hybridisiert. Nach Subklonierung wurde ein Klon erhalten, der das Gen für p100 enthält. Es wurde folgende kodierende DNA-Sequenz von p100 (5'-Ende) des Stammes PKo bei einer Länge von 346 Basenpaaren ermittelt: 
5' ATG AAA AAA ATG TTA CTA ATC TTT AGT TTT TTT CTT GTT
TTT TTA AAT GGA TTT CCT CTT AAT GCA AGG GAA GTT GAT AAG GAA
AAA TTA AAG GAC TTT GTT AAT ATG GAT CTT GAA TTT GTT AAT TAC
AAG GGT CCT TAT GAT TCT ACA AAT ACA TAT GAA CAA ATA GTA GGT
ATT GGG GAG TTT TTA GCA AGG CCG TTG ATC AAT TCC AAT AGT AAG
TCA AGT TAT TAT GGT AAA TAT TTT GTT AAT AGA TTT ATT GAC GAT
CAA GAT AAA AAA GCA AGT GTT GAT ATT TTT TCT ATT GGT AGT AAG
TCA GAG CTT GAT AGT ATA TTA AAT CTA AGA AGA ATT C... 3'
 Nach vollständiger Klonierung wurde die folgende Aminosäuresequenz ermittelt:

Met Lys Lys Met Leu Leu Ile Phe Ser Phe Phe Leu Val Phe Leu
Asn Gly Phe Pro Leu Asn Ala Arg Glu Val Asp Lys Glu Lys Leu
Lys Asp Phe Val Asn Met Asp Leu Glu Phe Val Asn Tyr Lys Gly
Pro Tyr Asp Ser Thr Asn Thr Tyr Glu Gln Ile Val Gly Ile Gly
Glu Phe Leu Ala Arg Pro Leu Ile Asn Ser Asn Ser Asn Ser Ser
Tyr Tyr Gly Lys Tyr Phe Val Asn Arg Phe Ile Asp Asp Gln Asp
Lys Lys Ala Ser Val Asp Ile Phe Ser Ile Gly Ser Lys Ser Glu
Leu Asp Ser Ile Leu Asn Leu Arg Arg Ile Leu Thr Gly Tyr Leu
Ile Lys Ser Phe Asp Tyr Glu Arg Ser Ser Ala Glu Leu Ile Ala
Lys Ala Ile Thr Ile Tyr Asn Ala Val Tyr Arg Gly Asp Leu Asp
Tyr Tyr Lys Glu Phe Tyr Ile Glu Ala Ser Leu Lys Ser Leu Thr
Lys Glu Asn Ala Gly Leu Ser Arg Val Tyr Ser Gln Trp Ala Gly
Lys Thr Gln Ile Phe Ile Pro Leu Lys Lys Asn Ile Leu Ser Gly
Asn Val Glu Ser Asp Ile Asp Ile Asp Ser Leu Val Thr Asp Lys
Val Val Ala Ala Leu Leu Ser Glu Asn Glu Ser Gly Val Asn Phe
Ala Arg Asp Ile Thr Asp Ile Gln Gly Glu Thr His Lys Ala Asp
Gln Asp Lys Ile Asp Ile Glu Leu Asp Asn Phe His Glu Ser Asp
Ser Asn Ile Thr Glu Thr Ile Glu Asn Leu Arg Asp Gln Leu Glu
Lys Ala Thr Asp Glu Glu His Lys Lys Glu Ile Glu Ser Gln Val
Asp Ala Lys Lys Lys Gln Lys Glu Glu Leu Asp Lys Lys Ala Ile
Asp Leu Asp Lys Ala Gln Gln Lys Leu Asp Phe Ala Glu Asp Asn
Leu Asp Ile Gln Arg Asp Thr Val Arg Glu Lys Leu Gln Glu Asn
Ile Asn Glu Thr Asn Lys Glu Lys Asn Leu Pro Lys Pro Gly Asp
Val Ser Ser Pro Lys Val Asp Lys Gln Leu Gln Ile Lys Glu Ser
Leu Glu Asp Leu Gln Glu Gln Leu Lys Glu Ala Ser Asp Glu Asn
Gln Lys Arg Glu Ile Glu Lys Gln Ile Glu Ile Lys Lys Asn Asp
Glu Glu Leu Phe Lys Asn Lys Asp His Lys Ala Leu Asp Leu Lys
Gln Glu Leu Asn Ser Lys Ala Ser Ser Lys Glu Lys Ile Glu Gly
Glu Glu Glu Asp Lys Glu Leu Asp Ser Lys Lys Asn Leu Glu Pro
Val Ser Glu Ala Asp Lys Val Asp Lys Ile Ser Lys Ser Asn Asn
Asn Glu Val Ser Lys Leu Ser Pro Leu Asp Glu Pro Ser Tyr Ser
Asp Ile Asp Ser Lys Glu Gly Val Asp Asn Lys Asp Val Asp Leu
Gln Lys Thr Lys Pro Gln Val Glu Ser Gln Pro Thr Ser Leu Asn
Glu Asp Leu Ile Asp Val Ser Ile Asp Ser Ser Asn Pro Val Phe
Leu Glu Val Ile Asp Pro Ile Thr Asn Leu Gly Thr Leu Gln Leu
Ile Asp Leu Asn Thr Gly Val Arg Leu Lys Glu Ser Ala Gln Gln
Gly Ile Gln Arg Tyr Gly Ile Tyr Glu Arg Glu Lys Asp Leu Val
Val Ile Lys Ile Asp Ser Gly Lys Ala Lys Leu Gln Ile Leu Asp
Lys Leu Glu Asn Leu Lys Val Ile Ser Glu Ser Asn Phe Glu Ile
Asn Lys Asn Ser Ser Leu Tyr Val Asp Ser Arg Met Ile Leu Val
Val Val Lys Asp Asp Ser Asn Ala Trp Arg Leu Ala Lys Phe Ser
Pro Lys Asn Leu Asp Glu Phe Ile Leu Ser Glu Asn Lys Ile Leu
Pro Phe Thr Ser Phe Ala Val Arg Lys Asn Phe Ile Tyr Leu Gln
Asp Glu Leu Lys Ser Leu Val Thr Leu Asp Val Asn Thr Leu Lys
Lys Val Lys

### Aminosäuresequenz des p100 Proteins

In analoger Weise wurden über die pC-Aminosäuresequenzen: pl: Lys Ile Thr Asp Ser Asn Ala Thr Val Leu Ala Val Lys p2: Asp Leu Phe Glu Ser Val Glu Gly Leu Leu Lys die entsprechenden Oligodesoxynukleotid-Sequenzen synthetisiert:
pC-p1-Oligodesoxynukleotid-Sequenz: 
AA(G;A) AT(T;A) AC(A;T) GA(T;C) (A;T)C(A;T) AA(T;C) GC(A;T)
AC(A;T) GT(A;T) (T;C)T(G;A;T) GC(A;T) GT(A;T) AA(A;G) A
pC-p2-Oligodesoxynukleotid-Sequenz:

GA(T;C) (C;T)T(G;A;T) TT(T;C) GA(G;A) (T;A)C(A;T) GT(A;T)
GA(G;A) GG(A;T;C) (T;C)T(G;A;T) (T;C)T(G;A;T) AA(A;G) A

Nach dem Auffinden von geeigneten Klonen durch Hybridisierung und Subklonierung des gewünschten Gens konnte folgende kodierende DNA-Sequenz von pC des Stammes PKo bei einer Länge von 639 Basenpaaren ermittelt werden:

5' ATG AAA AAG AAT ACA TTA AGT GCG ATA TTA ATG ACT TTA
TTT TTA TTT ATA TCT TGT AAT AAT TCA GGG AAG GTG GGG ATT CTG
CAT CTA CTA ATC CTG CTG ACG AGT CTT GCG AAA GGG CCT AAT CTT
ACA GAA ATA AGC AAA AAA ATT ACA GAT TCT AAT GCA TTT GTA CTT
GCT GTT AAA GAA GTT GAG ACT TTG GTT TTA TCT ATA GAT GAA CTT
GCT AAG AAA GCT ATT GGT CAA AAA ATA GAC AAT AAT AAT GGT TTA
GCT GCT TTA AAT AAT CAG AAT GGA TCG TTG TTA GCA GGA GCC TAT
GCA ATA TCA ACC CTA ATA ACA GAA AAA TTG AGT AAA TTG AAA AAT
TTA GAA GAA TTA AAG ACA GAA ATT GCA AAG GCT AAG AAA TGT TCC
GAA GAA TTT ACT AAT AAA CTA AAA AGT GGT CAT GCA GAT CTT GGC
AAA CAG GAT GCT ACC GAT GAT CAT GCA AAA GCA GCT ATT TTA AAA
ACA CAT GCA ACT ACC GAT AAA GGT GCT AAA GAA TTT AAA GAT TTA
TTT GAA TCA GTA GAA GGT TTG TTA AAA GCA GCT CAA GTA GCA CTA
ACT AAT TCA GTT AAA GAA CTT ACA AGT CCT GTT GTA GCA GAA AGT
CCA AAA AAA CCT TAA 3'

Das Protein pC weist bei einer Länge von 212 Aminosäuren folgende Sequenzen auf:

Met Lys Lys Asn Thr Leu Thr Ala Ile Leu Met Thr Leu Phe Leu
Phe Ile Ser Cys Asn Asn Ser Gly Lys Val Gly Ile Leu Thr Ser
Thr Asn Pro Ala Asp Glu Ser Ala Lys Gly Pro Asn Leu Thr Glu
Ile Ser Lys Lys Ile Thr Asp Ser Asn Ala Phe Val Leu Ala Val
Lys Glu Val Glu Thr Leu Val Leu Ser Ile Asp Glu Leu Ala Lys
Lys Ala Ile Gly Gln Lys Ile Asp Asn Asn Asn Gly Leu Ala Ala
Leu Asn Asn Gln Asn Gly Ser Leu Leu Ala Gly Ala Tyr Ala Ile
Ser Thr Leu Ile Thr Glu Lys Leu Ser Lys Leu Lys Asn Leu Glu
Glu Leu Lys Thr Glu Ile Ala Lys Ala Lys Lys Cys Ser Glu Glu
Phe Thr Asn Lys Leu Lys Ser Gly His Ala Asp Leu Gly Lys Gln
Asp Ala Thr Asp Asp His Ala Lys Ala Ala Ile Leu Lys Thr His
Ala Thr Thr Asp Lys Gly Ala Lys Glu Phe Lys Asp Leu Phe Glu
Ser Val Glu Gly Leu Leu Lys Ala Ala Gln Val Ala Leu Thr Asn
Ser Val Lys Glu Leu Thr Ser Pro Val Val Ala Glu Ser Pro Lys
Lys Pro

### Aminosäuresequenz des PC Proteins - 22kD -

In entsprechender Weise wurde auch ein Teil der kodierenden DNA-Sequenz von OspA (5'-Ende) des Stammes PKo bei einer Länge von 680 Basenpaare ermittelt:

5' ATG AAA AAA TAT TTA TTG GGA ATA GGT CTA ATA TTA GCC
TTA ATA GCA TGC AAG CAA AAT GTT AGC AGC CTT GAT GAA AAA AAC
AGC GCT TCA GTA GAT TTG CCT GGT GAG ATG AAA GTT CTT GTA AGT
AAA GAA AAA GAC AAA GAC GGT AAG TAC AGT CTA AAG GCA ACA GTA
GAC AAG ATT GAG CTA AAA GGA ACT TCT GAT AAA GAC AAT GGT TCT
GGG GTG CTT GAA GGT ACA AAA GAT GAC AAA AGT AAA GCA AAA TTA
ACA ATT GCT GAC GAT CTA AGT AAA ACC ACA TTC GAA CTT TTC AAA
GAA GAT GGC AAA ACA TTA GTG TCA AGA AAA GTA AGT TCT AAA GAC
AAA ACA TCA ACA GAT GAA ATG TTC AAT GAA AAA GGT GAA TTG TCT
GCA AAA ACC ATG ACA AGA GAA AAT GGA ACC AAA CTT GAA TAT ACA
GAA ATG AAA AGC GAT GGA ACC GGA AAA GCT AAA GAA GTT TTA AAA
AAC TTT ACT CTT GAA GGA AAA GTA GCT AAT GAT AAA GTA ACA TTG
GAA GTA AAA GAA GGA ACC GTT ACT TTA AGT AAG GAA ATT GCA AAA
TCT GGA GAA GTA ACA GTT GCT CTT AAT GAC ACT AAC ACT ACT CAG
GCT ACT AAA AAA ACT GGC GCA TGG GAT TCA AAA ACT TCT ACT TTA
ACA ATT AGT GT...3'

Nach vollständiger Sequenzierung konnte die folgende Aminosäuresequenz für das 31 kD Protein ermittelt werden:

Met Lys Lys Tyr Leu Leu Gly Ile Gly Leu Ile Leu Ala Leu Ile
Ala Cys Lys Gln Asn Val Ser Ser Leu Asp Glu Lys Asn Ser Ala
Ser Val Asp Leu Pro Gly Glu Met Lys Val Leu Val Ser Lys Glu
Lys Asp Lys Asp Gly Lys Tyr Ser Leu Lys Ala Thr Val Asp Lys
Ile Glu Leu Lys Gly Thr Ser Asp Lys Asp Asn Gly Ser Gly Val
Leu Glu Gly Thr Lys Asp Asp Lys Ser Lys Ala Lys Leu Thr Ile
Ala Asp Asp Leu Ser Lys Thr Thr Phe Glu Leu Phe Lys Glu Asp
Gly Lys Thr Leu Val Ser Arg Lys Val Ser Ser Lys Asp Lys Thr
Ser Thr Asp Glu Met Phe Asn Glu Lys Gly Glu Leu Ser Ala Lys
Thr Met Thr Arg Glu Asn Gly Thr Lys Leu Glu Tyr Thr Glu Met
Lys Ser Asp Gly Thr Gly Lys Ala Lys Glu Val Leu Lys Asn Phe
Thr Leu Glu Gly Lys Val Ala Asn Asp Lys Val Thr Leu Glu Val
Lys Glu Gly Thr Val Thr Leu Ser Lys Glu Ile Ala Lys Ser Gly
Glu Val Thr Val Ala Leu Asn Asp Thr Asn Thr Thr Gln Ala Thr
Lys Lys Thr Gly Ala Trp Asp Ser Lys Thr Ser Thr Leu Thr Ile
Ser Val Asn Ser Lys Lys Thr Thr Gln Leu Val Phe Thr Lys Gln
Asp Thr Ile Thr Val Gln Lys Tyr Asp Ser Ala Gly Thr Asn Leu
Glu Gly Thr Ala Val Glu Ile Lys Thr Leu Asp Glu Leu Lys Asn
Ala Leu Lys

### Aminosäuresequenz von OspA (Stamm Pko)

### Beispiel 4:

### Reinigung der rekombinant produzierten B. burgdorferi Antigene

### a) am Beispiel des p41 (Flagellin)

Eine 50 ml Ubernachtkultur des in Beispiel 2 beschriebenen Klons pUC8ly2 wurde in 1,5 ml frisches L-Broth Medium gegeben und bei 37°C und intensiven Schütteln inkubiert. Bei Erreichen einer optischen Dichte von 0,7 wurde die Kultur mit IPTG in einer Endkonzentration von 1 mM induziert und für weitere 3 h inkubiert. Die Bakterien wurden pelletiert (6000 rpm. 10 min.), in 300 ml 20 mM Tris-HCl pH 8,0, 50 mM EDTA, 0,5 mg/ml Lysozym resuspendiert und für 45 min. in ein 37°C Wasserbad gegeben. Nach der Zugabe von NaCl in einer Endkonzentration von 150 mM und Triton-X-100 in einer Endkonzentration von 1 % wurde weiter für 45 min. bei 37°C inkubiert, und die Suspension anschließend dreimal für je 5 min. mit Ultraschall behandelt. Unlösliche Bestandteile wurden bei 9000 rpm in 30 min. pelletiert, in 20 mM Tris-HCl pH 8,0, 10 mM Dithiothreitol und 1 % Octyl-gluco-pyranosid (Sigma-Chemie, München) resuspendiert und für 1 h bei Raumtemperatur gerührt. Nach der anschließenden Pelletierung unlöslicher Bestandteile bei 17000 rpm in 30 min. wurde der Überstand vorsichtig dekantiert.

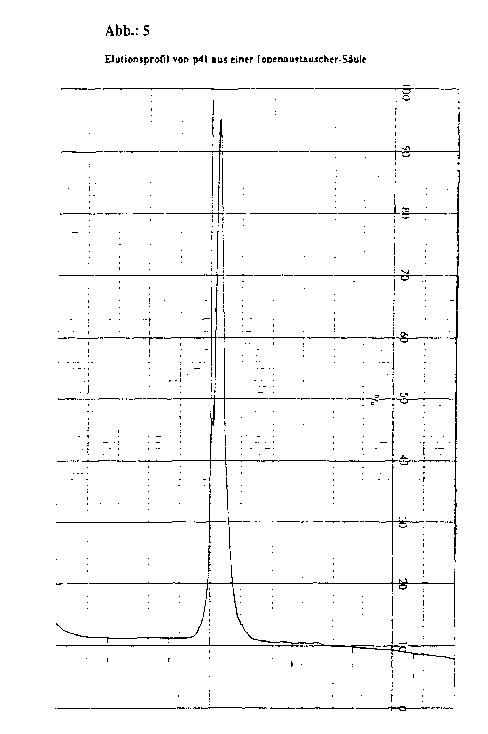

Anschließend wurde das Pellet in 150 ml 20 mM Tris-HCl pH 8,0, 8 M Harnstoff, 1 % β-Mercaptoethanol durch Rühren für 2h resuspendiert. Auch hier unlösliche Bestandteile wurden erneut durch Zentrifugation bei 17000 rpm in 30 min. abgetrennt, und der Überstand auf eine DEAE-Sephacel-Säule (Pharmacia, Freiburg) mit einem Gelvolumen von 550ml (3cm Durchmesser, 80cm Höhe) gepumpt. Die Elution des p41 Antigens erfolgte in einem NaCl-Gradienten von 0-800 mM in einem Gesamtvolumen von 600 ml. Das rekombinante p41 wird bei einer NaCl-Konzentration von etwa 0,25 M eluiert. Die entsprechenden Fraktionen wurden vereinigt und weiter durch eine HPLC mit einer Mono Q Säule (Anionenaustauscher) aufgereinigt (Abb.4). Ein Elutionsprofil mit dem gereinigten p41 in einem NaCl-Gradienten von 0-800 mM ist in Abb.5 gezeigt. Die hier p41-positiven Fraktionen (nach Western Blot Analyse) wurden gegen 20 mM Tris-HCl pH 8,0, 10 mM MgCl₂ und 0,1 % β-Mercaptoethanol dialysiert und anschließend für die in Beispiel 5 gezeigten Teste verwendet. Aus einer Reinigung von p41 ausgehend von 11 Bakterienkultur kann typischerweise eine Ausbeute von 5 bis 10mg erwartet werden.

Nach Sequenzierung konnte die folgende Aminosäuresequenz ermittelt werden: 
Met Arg Gly Ser Ile Met Ile Ile Asn His Asn Thr Ser Ala Ile
Asn Ala Ser Arg Asn Asn Ala Ile Asn Ala Ala Asn Leu Ser Lys
Thr Gln Glu Lys Leu Ser Ser Asn Tyr Arg Ile Asn Arg Ala Ser
Asp Asp Ala Ala Gly Met Gly Val Ser Gly Lys Ile Asn Ala Gln
Ile Arg Gly Leu Ser Gln Ala Ser Arg Asn Thr Ser Lys Ala Ile
Asn Phe Ile Gln Thr Thr Glu Gly Asn Leu Asn Glu Val Glu Lys
Val Leu Val Arg Met Lys Glu Leu Ala Val Gln Ser Gly Asn Gly
Thr Tyr Ser Asp Ser Asp Arg Gly Ser Ile Gln Ile Glu Ile Glu
Gln Leu Thr Asp Glu Ile Asn Arg Ile Ala Asp Gln Ala Gln Tyr
Asn Gln Met His Met Leu Ser Asn Lys Ser Ala Ser Gln Asn Val
Lys Thr Ala Glu Glu Leu Gly Met Gln Pro Ala Lys Ile Asn Thr
Pro Ala Ser Leu Ser Gly Ser Gln Ala Ser Trp Thr Leu Arg Val
His Val Gly Ala Asn Gln Asp Glu Ala Ile Ala Val Asn Ile Tyr
Ser Ala Asn Val Ala Asn Leu Phe Ala Gly Glu Gly Ala Gln Ala
Ala Gln Ala Ala Pro Val Gln Glu Gly Ala Gln Glu Glu Gly Ala
Gln Gln Pro Thr Pro Ala Thr Ala Pro Thr Gln Gly Gly Val Asn
Ser Pro Val Asn Val Thr Thr Thr Val Asp Ala Asn Thr Ser Leu
Ala Lys Ile Glu Asn Ala Ile Arg Met Ile Ser Asp Gln Arg Ala
Asn Leu Gly Ala Phe Gln Asn Arg Leu Glu Ser Ile Lys Asn Ser
Thr Glu Tyr Ala Ile Glu Asn Leu Lys Ala Ser Tyr Ala Gln Ile
Lys Asp Ala Thr Met Thr Asp Glu Val Val Ala Ala Thr Thr Asn
Ser Ile Leu Thr Gln Ser Ala Met Ala Met Ile Ala Gln Ala Asn
Gln Val Pro Gln Tyr Val Leu Ser Leu Leu Arg
 Aminosäuresequenz des p41 Proteins

### b) Reinigung von rekombinantem Borrelia burgdorferi Antigen pC aus E.coli

Ein Klon, der das Gen für das Antigen pC enthält. (pDSIPC5) wird in 100ml L-Broth (mit 50 µg Ampicillin/ml) angeimpft, über Nacht wachsen gelassen und dann in 900ml L-Broth/Ampicillin - doppelt konzentrierter Hefeextrakt/2ml Glycerin - überführt, nach ca. 1h mit 2mM IPTG induziert und 2 - 3h weiter geschüttelt.

Nach Abzentrifugieren bei 8000 Upm für 10 min. wird das Pellet in 20ml Lyse-Puffer (50mM Tris-HCl, pH 7,5, 2mM EDTA, 0.1mM DTE, 0.1mM PMSF; 0.4 mg/ml Lysozym) resuspendiert. Nach 30 min. Rühren bei Raumtemperatur erfolgt Zugabe von Triton-X 100 (Endkonzentration 0.1 - 0.2 %). Zusätzlich werden 10 µl Benzonase (Merck) zugegeben. Es wird weitere 30 min. bei Raumtemperatur gerührt. Die jetzt klare Suspension wird mit festem NaCl auf 1M NaCl eingestellt und weitere 30 min. - 60 min. gerünrt (bei 4°C).

Nach Zentrifugation bei 4°C, 30 min., 15.000 Upm befindet sich das pC-Protein quantitativ im Überstand. Das Pellet wird verworfen. Der Überstand wird gegen 10mM Tris-HCl, pH 8.0 bei mehrmaligem Pufferwechsel dialysiert. Nach Zentrifugation und/oder Filtration wird auf DEAE-Sepharose (Pharmacia) aufgetragen, wobei die Säule mit 10mM Tris-HCl, pH 8.0 equilibriert wird. Bei Elution mit 0 M NaCl befindet sich das pC-Protein im 2. Peak des Durchlaufs. Die ersten Fraktionen können verworfen werden, der Rest wird gesammelt und rechromatographiert. Die Trennsäule wird mit 1 M NaCl regeneriert und in 10mM Tris-HCl pH 8.0 equilibriert. Das so erhaltene Antigen kann nun in einem geeigneten Testkit bspw. einem ELISA verwendet werden.

### c) Reinigung von rekombinantem Barreila burgdorferi Antigen OspA aus E.coll

Ein Klon, der das Gen für das Antigen OspA enthält (pDSIOspA) wird in 100ml L-Broth (mit 50 µg Ampicillin/ml) angeimpft und über Nacht angezogen. Die Kulturbrühe wird in 900ml L-Broth/Ampicillin - doppelt konzentrierter Hefeextrakt/2ml Glycerin - überführt, nach ca. 1h mit 2mM IPTG induziert und 2 - 3h weiter geschüttelt.

Die Zellen werden bei 6000Upm, 5min. abzentrifugiert und das Pellet in 20ml Lyse-Puffer (50mM Tris-HCl, pH 7,5, 2mM EDTA, 0,1mM DTE, 0,1mM PMSF; 0,4mg/ml Lysozym) resuspendiert. Nach 30min. Rühren bei Raumtemperatur erfolgt Zugabe von Triton-X 100 (Endkonzentration 0,5 - 1 %). Zusätzlich werden 10 µl Benzonase (MERCK) zugegeben. Darauf wird weitere 30min. bei Raumtemperatur gerührt.

Die jetzt klare Suspension wird mit festem NaCl auf 1 M NaCl eingestellt und weiter gerührt (bei 4°C). Nach Zentrifugation bei 4°C für 30min. und 15.000Upm befindet sich OspA beinahe quantitativ im Pellet. Der Überstand wird verworfen und das Pellet in 2M Harnstoff (mit 50mM Tris-HCl, pH 7,5, 2mM EDTA, 0,1mM DTE) resuspendiert. OspA ist nun im Überstand.

Der Überstand wird im Dialyse-Schlauch gegen 5mM MES (2-[N-Morpholino]ethan-sulfonsäure) Puffer, pH 6.0 dialysiert, wobei ein mehrmaliger Pufferwechsel unbedingt erforderlich ist. Nach Zentrifugation und Filtration wird das Protein auf S-Sepharose fast flow (Pharmacia) Säule aufgetragen. Zuerst wird mit 0 M NaCl gewaschen, dann mit einem Gradienten von 0 bis 1 M NaCl eluiert. Das OspA-Antigen eluiert als scharfer Peak bei etwa 0,4 M NaCl. Nach Dialyse gegen 10 mM-Tris-HCl pH 7,5 kann das OspA Antigen in einem geeigneten Testkit, bspw. einem ELISA verwendet werden.

### Beispiel 5:

### Einsatz rekombinant produzierter B. burgdorferi Antigene (Beispiel p41) im ELISA

Bedingt durch die hohe Reinheit der produzierten rekombinanten Antigene sind B. burgdorferi spezifische Teste möglich, die maschinenlesbar und ohne großen technischen und personellen Aufwand durchführbar sind.

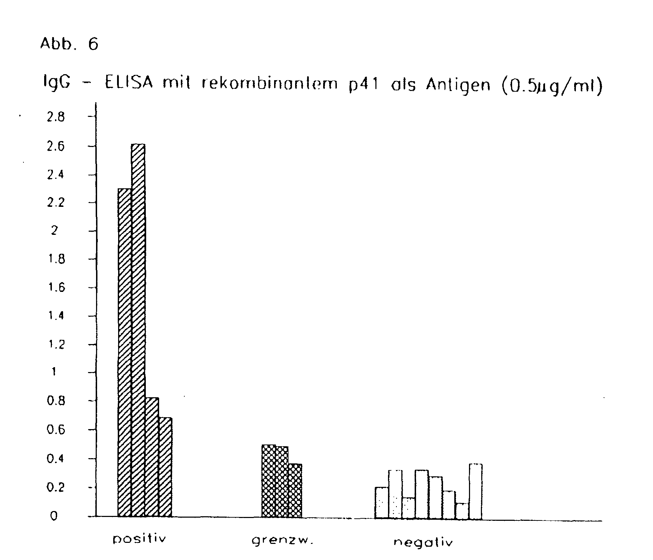

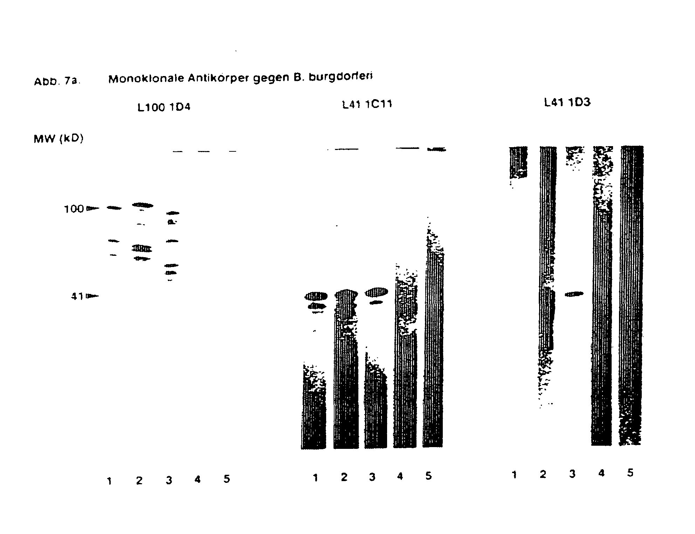

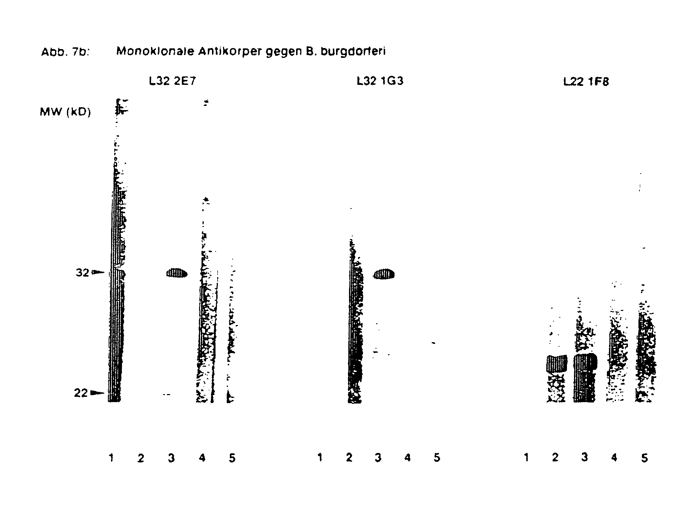

Mikrotiterplatten wurden mit je 50µl des gereinigten p41 (Konzentration 0,5 - 5 µg/ml) pro Napf beschichtet. Die Platten wurden nach Standardmethoden bei 4°C über Nacht inkubiert, gewaschen und die noch freien Bindungsstellen 2 %iger Rinderserum-Albumin-Lösung abgesättigt. Anschließend wurden jeweils 50 µl Serum (Verdünnung 1:200) dazupipettiert, für 2 h bei 37°C inkubiert, ungebundene Teile abgewaschen und die gebundenen Immunkomplexe mit je 50 µl Peroxidasemarkiertem anti-human IgG (Verdünnung 1:1000) detektiert. Nach abermaligen Waschen wurden die Näpfe mit je 100µl ortho-Phenylendiamin (Konzentration 0,1 % in 0,1 M Phosphatpuffer pH 6,0 mit 0,03 % H₂O₂) als Färbereagens beschickt und die im Dunkeln durchgeführte Färbung nach 10 min. mit 100 µl 1 N Schwefelsäure gestoppt. Die Mikrotiterplatte wurde in einem Photometer bei 486 nm ausgewertet (Abb. 6).

In dem hier gezeigten Beispiel wurden 7 positive und 8 negative anti-B. burgdorferi Seren ausgetestet. Von den klinisch gesicherten Lyme-positiven Seren waren drei, die auf Western Blot Streifen mit B. burgdorferi als Antigen keine Reaktion mit p41 zeigten, d.h. Seren aus dem Frühstadium der Infektion darstellten. Diese reagierten im ELISA mit dem rekombinanten Antigen ebenfalls nur grenzwertig. Normal p41-positive Seren dagegen reagierten sehr gut, während Lymenegative Seren im Bereich von unter einer OD = 0,3 blieben.

### Beispiel 6:

### Herstellung B. burgdorferi spezifischer monoklonaler Antikörper

Weibliche Balb/c Mäuse wurden mit B. burgdorferi (DSM-Nr. 5662) intraperitonal immunisiert. Die 1. Immunisierung erfolgte mit kompletten Freund'schen Adjuvans, 2-5 weitere Immunisierungen mit inkomplettem Freund'schen Adjuvans folgten im Abstand von 2 Wochen. 2 Wochen später wurde das Antigen ohne Adjuvans appliziert und 3 Tage später die Mäuse getötet und die Milz entfernt.

Die Milz-Lymphozyten wurden mit Maus-Myelom-Zellen (Ag8-653) im Verhältnis 1:1 gemischt, sedimentiert und mit Fusionslösung (2,5 g Polyethylenglykol (PEG), 2,5 ml RPMI-Medium, 250 µl DMSO) versetzt: 1 min. Zugabe der Fusionslösung, 90 sek. Inkubation bei 37°C. Die Zellen wurden erneut sedimentiert, das PEG entfernt und Kulturmedium (HAT-Medium) hinzugegeben. Schließlich wurde die Zellsuspension in Mikrotiterplatten, die Makrophagen als Feederzellen enthielten, ausgesät und bebrütet. Hybridoma-Überstände wurden unverdünnt im indirekten Immunfluorezenz (IFT) getestet (Wilske, B.; Schierz, G.; Preac-Mursic, V.; Weber, K; Pfister, H.-W.; Einhäupl, K. (1984): Serological diagnosis of Erythema migrans disease and related disorders. Infection,12. 331-337).

IFT-positive Zellüberstände wurden mittels Western Blot analysiert. Im Western Blot reaktive Hybridomas wurden 4 mal mittels "limiting dilution" subkloniert und bezüglich ihrer Immunglobulin-Klasse und IgG-Subklasse identifiziert.

Auf diese Weise wurden folgende monoklonale Antikörper (MAB) erhalten:
1. MAB gegen p41:
   (a) L41 1C11
      Dieser Antikörper war mit allen 30 getesteten B. burgdorferi Stämmen und mit Rückfallfieber Borrelien (außer B. hermsii), nicht jedoch mit Treponemen reaktiv.
   (b) L41 1D3
      Dieser Antikörper war mit der Mehrzahl (21 von 24) der B. burgdorferi Stämme, nicht jedoch mit Rückfallfieber Borrelien und Treponemen reaktiv.
2. MAB gegen p100 (L 100 1D4): Dieser Antikörper war mit allen 30 getesteten B. burgdorferi Stämmen, nicht jedoch mit Rückfallfieber Borrelien oder Treponemen reaktiv.
3. MAB gegen pC (L22 1F8): Dieser MAB war mit pC-Proteinen von Haut- und Liquorstämmen reaktiv, dagegen waren die pC-Proteine einiger, aber nicht aller Zeckenstämme negativ.
4. MAB gegen OspA:
   OspA ist ein Hauptprotein (30 kD-Bereich) der äußeren Membran der meisten B. burgdorferi Stämme. OspA Proteine europäischer B. burgdorferi Stämme sind antigenetisch heterogen und unterscheiden sich antigenetisch von den amerikanischen Stämmen. Die wenigen OspA-negativen Stämme haben pC-Proteine.
   (a) L 32 2E7
      Insgesamt waren 29 von 32 Stämmen reaktiv. Die negativen Stämme wiesen kein OspA-Protein auf. Die 3 negativen Stämme waren mit dem pC-spezifischen MAB L22 1F8 reaktiv.
   (b) L32 1 G3:
      Dieser MAB war nur bei 3 von 25 getesteten Stämmen reaktiv.

Die Kombination von MAB L32 2E7 und MAB L22 1F8 sowie die Reaktion mit MAB L100 1D4 erlaubt die Identifizierung von B. burgdorferi Borrelien und Treponemen. Eine sichere Identifizierung und Differenzierung von B. burgdorferi war mit bisher verfügbaren monoklonalen Antikörpern nicht möglich.

### Beispiel 7:

### Ermittlung der Aminosäuresequenz eines Proteins mit einem Molekulargewicht von etwa 22kD aus einem anderen Stamm

Gemäß dem in den vorstehenden Beispielen beschriebenen Methoden wurde die Aminosäuresequenz eines Proteins mit einem Molekulargewicht von etwa 22 kD ermittelt. Dieses Protein wurde aus einem anderen Borrelien-Stamm kloniert und anschließend sequenziert. Dieser Stamm wurde bei der ATCC unter der Nummer 35210 hinterlegt und ist allgemein zugänglich. Dabei wurde folgende Aminosäuresequenz ermittelt:

Met Lys Lys Asn Thr Leu Ser Ala Ile Leu Met Thr Leu Phe Leu
Phe Ile Ser Cys Asn Asn Ser Gly Lys Asp Gly Asn Thr Ser Ala
Asn Ser Ala Asp Glu Ser Val Lys Gly Pro Asn Leu Thr Glu Ile
Ser Lys Lys Ile Thr Asp Ser Asn Ala Val Leu Leu Ala Val Lys
Glu Val Glu Ala Leu Leu Ser Ser Ile Asp Glu Ile Ala Ala Lys
Ala Ile Gly Lys Lys Ile His Gln Asn Asn Gly Leu Asp Thr Glu
Asn Asn His Asn Gly Ser Leu Leu Ala Gly Ala Tyr Ala Ile Ser
Thr Leu Ile Lys Gln Lys Leu Asp Gly Leu Lys Asn Glu Gly Leu
Lys Glu Lys Ile Asp Ala Ala Lys Lys Cys Ser Glu Thr Phe Thr
Asn Lys Leu Lys Glu Lys His Thr Asp Leu Gly Lys Glu Gly Val
Thr Asp Ala Asp Ala Lys Glu Ala Ile Leu Lys Thr Asn Gly Thr
Lys Thr Lys Gly Ala Glu Glu Leu Gly Lys Leu Phe Glu Ser Val
Glu Val Leu Ser Lys Ala Ala Lys Glu Met Leu Ala Asn Ser Val
Lys Glu Leu Thr Ser Pro Val Val Ala Glu Ser Pro Lys Lys Pro

### Aminosäuresequenz von PC Protein

### Beispiel 8:

### Vergleich von Testkits mit erfindungsgemäßen Proteinen gegenüber solchen, bei denen ein Ultrasonikat verwendet wurde

Es wurden 74 Seren von Patienten mit Erythema migrans auf IgM- und IgG-Antikörper getestet. Zusätzlich wurde eine negative Kontrollgruppe von 100 Blutspendern überprüft. Bei den Tests wurden gemäß an sich bekannten ELISA-Testdurchführungsmethoden einmal Ultrasonikatpräparationen von Borrelia burgdorferi eingesetzt. Andererseits wurden erfindungsgemäß hergestellte rekombinante Proteine getrennt und gemeinsam eingesetzt. Die nachfolgenden Tabellen zeigen eindeutig, daß durch das erfindungsgemäße Verfahren eine beträchtlich höhere Sensitivität erreicht werden kann im Vergleich zu dem Einsatz von Ultrasonikat.

| NACHWEIS von IgM-Antikörpern | |
|---|---|
| ELISA/Antigen | Erythema migrans (n=74) |
| Ultrasonikat | 20 27.0% |
| | |
| p41 (recomb.) | 2229.7% |
| OspA(rekomb.) | 79.4% |
| pC(rekomb.) | 2635.1% |
| | |
| | |
| p41 und/oder pC | 34 45.9% |
| p41 und/oder pC und/oder OspA | 3445.9% |

| NACHWEIS von IgG-Antikörpern | |
|---|---|
| ELISA/Antigen | Erythema migrans (n=74) |
| Ultrasonikat | 17 22.9% |
| | |
| p41(recomb.) | 23 31.1% |
| OspA(rekomb.) | 68.1% |
| pC(rekomb.) | 27 36.5% |
| | |
| p41 und/oder pC | 34 45.9% |
| p41 und/oder pC und/oder OspA | 35 47.3% |

| NACHWEIS von IgG-und/oder IgM-Antikörpern | |
|---|---|
| ELISA/Antigen | Erythema migrans (n=74) |
| Ultrasonikat | 30 40% |
| | |
| p41 (recomb.) | 39 53% |
| OspA(rekomb.) | 11 15% |
| pC(rekomb.) | 41 55% |
| | |
| p41 und/oder pC | 53 72% |
| p41 und/oder pC und/oder OspA | 53 72% |

### Beschreibung der Abbildungen

### Abb. 1 a und b:

### Reaktivität von B.burgdorferi-infizierten Patienten mit Lysaten von 5 verschiedenen B. burgdorferi Stämmen im Western Blot.

Getestet wurden Seren der Stadien II und III (Neuroborreliose, Stadium II (IgM und IgG); Acrodermatitis (lgG) und Arthritis (IgG), Stadium III). Die frühe Immunantwort ist unabhängig vom Teststamm gegen ein enges Spektrum von Borrelienproteinen gerichtet (pC und p41). Die späte Immunantwort ist gegen ein breites Panel von Borrelienproteinen gerichtet. immundominante Proteine sind (unabhängig vom Teststamm) p100 (mit variablen Molekulargewicht) und p41.

### Abb. 2

### 2a) Verlaufskontrolle (IgM-Westernblot) bei Erythema migrans

Das pC-Protein kann das immundominante Protein der frühen Immunantwort sein. Antikörper gegen p41 können später auftreten und nur schwach ausgeprägt sein. Bei längerer Krankheitsdauer können auch IgM-Antikörper gegen p17 auftreten.

### 2b) IgG-Western Blot bei Spätmanifestationen

IgG-Antikörper erkennen ein breites Spektrum von Borrelienproteinen. Bei Verwendung des Stammes PKo erweisen sich als immundominante Proteine p17 und p100. p17 wird vom Stamm PKo stark ausgeprägt (im Gegensatz zu anderen Stämmen; siehe Abb.1). Das Flagellin p41 wurde in 2 dieser Beispiele (Serum 1 und 2) nicht erkannt.

### Abb.3

### Schema der DNA-Amplifizierung der p41-kodierenden Region.

A: Ausschnitt der B.burgdorferi-DNA mit der p41-kodierenden Region (schwarzer Balken).
B: Vergrößerung des 5' bzw 3' Endes des p41-Gens mit des jeweiligen DNA-Sequenzen. Angegeben ist zusätzlich der Translationsstart (ATG) sowie das Stop-Codon am 3'-Ende (TAA). Die für die PCR benutzten Primer-Sequenzen sind zusätzlich unter (Primer 1) bzw über (Primer 2) dem p41 kodierende DNA-Doppelstrang angegeben. Eine Anhybridisierung der Primer kann nur mit den jeweiligen 3'-Bereichen erfolgen. Die 5'-Enden enthalten nicht-hybridisierende Teile, die Schnittstellen für Restriktions-Enzyme darstellen: GGATCC - BamHl; TCATGA - BspHl, am 5'- Ende; GACGTC - Pstl am 3'-Ende.

### Abb. 4

### Expression, Reaktivität und Reinigung von rekombinantem p41.

linker Teil: Coomassie-blau gefärbtes SDS-Polyacryamidgel. Die einzelnen Spuren waren wie folgt beladen: 1, E. coli-Lysat, negative Kontrolle; 2, E.coli-Lysat mit pUC81y17 nach IPTG-Induktion, das rekombinant produzierte p41 ist als zusätzliche Bande im Bereich von ca 45kDa zu erkennen; 3, Überstand des Lysats aus 2 nach Aufbrechen der Zellen wie in Beispiel 4 beschrieben; 4, Pellet-Fraktion der lysierten Zellen mit dem rekombinanten p41; 5, Octyl-gluco-pyranosid Überstand; 6, wie 5, jedoch Pellet-Fraktion; 7 - 10, Fraktionen nach Elution von p41 von einer MonoQ-Säule durch einen Salzgradienten; Spuren 9 und 10 enthalten rekombinantes p41, bedingt durch Degradationserscheinungen sowie unvollständige Translation treten neben dem vollständigen Produkt noch kleiner Fragmente auf, die sich jedoch auch in authentischen p41-Material aus B.burgdorferi finden lassen.

rechter Teil: Immun-gefärbter Western Blot eines SDS-Geles mit Proben des Coomassie-gefärbten Gels. Die Immunfärbung wurde mit einem in Beispiel 6 beschriebenen monoklonalen Antikörper durchgeführt. Bezeichung der Spuren bzw der Proben wie Coomassie-gefärbtes Gel: Spur 0, Leerspur.

### Abb. 5

### HPLC-Elutionsprofil von p41 aus einer lonenaustauscher·Säule mit einem Salsgradient.

Im Anschluß an die Anionenaustauscher-Reinigung (MonoQ von Pharmacia) von p41 wurde das Antigen gegen 4M Hamstoff ohne Salz zurückdialysiert und wiederum auf die MonoQ-Säule gegeben, um die Reinheit zu überprüfen. Das Elutionsprofil zeigt nur noch einen Proteinadsorptionsgipfel. Der kleinere Gipfel unmittelbar vor dem Hauptanteil entspricht dem in Abb. 4, Spur 8 sichtbaren p41-Fragment mit einer Größe von ca 30 kD (getestet durch Western Blot).

### Abb. 6:

### IgG-ELISA mit rekombinantem p41 als Antigen.

Das über Anionenaustauscher (MonoQ) gereinigte rekombinante Antigen (s. Abb. 5) wurde in einer Konzentration von 0,5 µg/ml eingesetzt. Es wurden 7 Seren von Patienten mit einer klinisch definierten Lyme Borreliose und 8 Seren von Gesunden getestet. 4 Seren der Lyme Borreliose Patienten waren im Western Blot stark reaktiv mit dem rekombinanten p41 (= positiv), 3 Seren schwach reaktiv (= grenzwertig), die Seren der Gesunden reagierten nicht (= negativ). Der IgG-ELISA zeigte ein vergleichbares Ergebnis. Y-Achse: Optische Dichte bei Wellenlänge 486 nm; grenzw. = grenzwertig

### Abb. 7

### Reaktivität von monoklonalen Antikörpern gegen verschiedene B.burgdorferi Antigene.

Sechs monoklonale Antikörper gegen B. burgdorferi wurden mit 30 verschiedenen B. burgdorferi-Stämmen, 4 Rückfallfieberborrelien-Stämmen und 2 verschiedenen Treponemen getestet. in der Abbildung sind drei verschiedene B. burgdorferi Isolate (1=B31. amerikan. Stamm; 2=PKo, deutscher Hautstamm: 3=PBi, deutscher Liquorstamm), eine Rückfallfieberborrelie (4=B. hermsii) und ein Treponemenstamm (5=T. phagedenis) exemplarisch dargestellt. Es wurden die gemäß Beispiel 6 hergestellten monoklonalen Antikörper eingesetzt.

## Patentansprüche (Patentansprüche für folgende(n) Vertragsstaat(en): AT, BE, CH, DE, DK, FR, GB, IT, LI, LU, NL, SE)

1. Immunologisch aktives Protein von Borrelia burgdorferi, das in einer von anderen aus Borrelia burgdorferi stammenden Proteinen freien Form vorliegt, und gentechnologisch hergestellt wurde, **dadurch gekennzeichnet, daß** es
a) die Aminosäuresequenz 
Met Lys Lys Asn Thr Leu Thr Ala Ile Leu Met Thr Leu Phe Leu
Phe Ile Ser Cys Asn Asn Ser Gly Lys Val Gly Ile Leu Thr Ser
Thr Asn Pro Ala Asp Glu Ser Ala Lys Gly Pro Asn Leu Thr Glu
Ile Ser Lys Lys Ile Thr Asp Ser Asn Ala Phe Val Leu Ala Val
Lys Glu Val Glu Thr Leu Val Leu Ser Ile Asp Glu Leu Ala Lys
Lys Ala Ile Gly Gln Lys Ile Asp Asn Asn Asn Gly Leu Ala Ala
Leu Asn Asn Gln Asn Gly Ser Leu Leu Ala Gly Ala Tyr Ala Ile
Ser Thr Leu Ile Thr Glu Lys Leu Ser Lys Leu Lys Asn Leu Glu
Glu Leu Lys Thr Glu Ile Ala Lys Ala Lys Lys Cys Ser Glu Glu
Phe Thr Asn Lys Leu Lys Ser Gly His Ala Asp Leu Gly Lys Gln
Asp Ala Thr Asp Asp His Ala Lys Ala Ala Ile Leu Lys Thr His
Ala Thr Thr Asp Lys Gly Ala Lys Glu Phe Lys Asp Leu Phe Glu
Ser Val Glu Gly Leu Leu Lys Ala Ala Gln Val Ala Leu Thr Asn
Ser Val Lys Glu Leu Thr Ser Pro Val Val Ala Glu Ser Pro Lys
Lys Pro
 oder eine Teilsequenz davon von wenigstens 10 Aminosäuren aufweist oder
b) die Aminosäuresequenz 
Met Lys Lys Asn Thr Leu Ser Ala Ile Leu Met Thr Leu Phe Leu
Phe Ile Ser Cys Asn Asn Ser Gly Lys Asp Gly Asn Thr Ser Ala
Asn Ser Ala Asp Glu Ser Val Lys Gly Pro Asn Leu Thr Glu Ile
Ser Lys Lys Ile Thr Asp Ser Asn Ala Val Leu Leu Ala Val Lys
Glu Val Glu Ala Leu Leu Ser Ser Ile Asp Glu Ile Ala Ala Lys
Ala Ile Gly Lys Lys Ile His Gln Asn Asn Gly Leu Asp Thr Glu
Asn Asn His Asn Gly Ser Leu Leu Ala Gly Ala Tyr Ala Ile Ser
Thr Leu Ile Lys Gln Lys Leu Asp Gly Leu Lys Asn Glu Gly Leu
Lys Glu Lys Ile Asp Ala Ala Lys Lys Cys Ser Glu Thr Phe Thr
Asn Lys Leu Lys Glu Lys His Thr Asp Leu Gly Lys Glu Gly Val
Thr Asp Ala Asp Ala Lys Glu Ala Ile Leu Lys Thr Asn Gly Thr
Lys Thr Lys Gly Ala Glu Glu Leu Gly Lys Leu Phe Glu Ser Val
Glu Val Leu Ser Lys Ala Ala Lys Glu Met Leu Ala Asn Ser Val
Lys Glu Leu Thr Ser Pro Val Val Ala Glu Ser Pro Lys Lys Pro
 oder eine Teilsequenz davon von wenigstens 10 Aminosäuren aufweist oder
c) aus der Aminosäuresequenz 
Met Arg Gly Ser Ile Met Ile Ile Asn His Asn Thr Ser Ala Ile
Asn Ala Ser Arg Asn Asn Ala Ile Asn Ala Ala Asn Leu Ser Lys
Thr Gln Glu Lys Leu Ser Ser Asn Tyr Arg Ile Asn Arg Ala Ser
Asp Asp Ala Ala Gly Met Gly Val Ser Gly Lys Ile Asn Ala Gln
Ile Arg Gly Leu Ser Gln Ala Ser Arg Asn Thr Ser Lys Ala Ile
Asn Phe Ile Gln Thr Thr Glu Gly Asn Leu Asn Glu Val Glu Lys
Val Leu Val Arg Met Lys Glu Leu Ala Val Gln Ser Gly Asn Gly
Thr Tyr Ser Asp Ser Asp Arg Gly Ser Ile Gln Ile Glu Ile Glu
Gln Leu Thr Asp Glu Ile Asn Arg Ile Ala Asp Gln Ala Gln Tyr
Asn Gln Met His Met Leu Ser Asn Lys Ser Ala Ser Gln Asn Val
Lys Thr Ala Glu Glu Leu Gly Met Gln Pro Ala Lys Ile Asn Thr
Pro Ala Ser Leu Ser Gly Ser Gln Ala Ser Trp Thr Leu Arg Val
His Val Gly Ala Asn Gln Asp Glu Ala Ile Ala Val Asn Ile Tyr
Ser Ala Asn Val Ala Asn Leu Phe Ala Gly Glu Gly Ala Gln Ala
Ala Gln Ala Ala Pro Val Gln Glu Gly Ala Gln Glu Glu Gly Ala
Gln Gln Pro Thr Pro Ala Thr Ala Pro Thr Gln Gly Gly Val Asn
Ser Pro Val Asn Val Thr Thr Thr Val Asp Ala Asn Thr Ser Leu
Ala Lys Ile Glu Asn Ala Ile Arg Met Ile Ser Asp Gln Arg Ala
Asn Leu Gly Ala Phe Gln Asn Arg Leu Glu Ser Ile Lys Asn Ser
Thr Glu Tyr Ala Ile Glu Asn Leu Lys Ala Ser Tyr Ala Gln Ile
Lys Asp Ala Thr Met Thr Asp Glu Val Val Ala Ala Thr Thr Asn
Ser Ile Leu Thr Gln Ser Ala Met Ala Met Ile Ala Gln Ala Asn
Gln Val Pro Gln Tyr Val Leu Ser Leu Leu Arg
 oder der Teilsequenz dieses Proteins mit einer Deletion von 20-25 Aminosäuren am N-Terminus des Proteins besteht, oder
d) die Aminosäuresequenz 
Met Lys Lys Met Leu Leu Ile Phe Ser Phe Phe Leu Val Phe Leu
Asn Gly Phe Pro Leu Asn Ala Arg Glu Val Asp Lys Glu Lys Leu
Lys Asp Phe Val Asn Met Asp Leu Glu Phe Val Asn Tyr Lys Gly
Pro Tyr Asp Ser Thr Asn Thr Tyr Glu Gln Ile Val Gly Ile Gly
Glu Phe Leu Ala Arg Pro Leu Ile Asn Ser Asn Ser Asn Ser Ser
Tyr Tyr Gly Lys Tyr Phe Val Asn Arg Phe Ile Asp Asp Gln Asp
Lys Lys Ala Ser Val Asp Ile Phe Ser Ile Gly Ser Lys Ser Glu
Leu Asp Ser Ile Leu Asn Leu Arg Arg Ile Leu Thr Gly Tyr Leu
Ile Lys Ser Phe Asp Tyr Glu Arg Ser Ser Ala Glu Leu Ile Ala
Lys Ala Ile Thr Ile Tyr Asn Ala Val Tyr Arg Gly Asp Leu Asp
Tyr Tyr Lys Glu Phe Tyr Ile Glu Ala Ser Leu Lys Ser Leu Thr
Lys Glu Asn Ala Gly Leu Ser Arg Val Tyr Ser Gln Trp Ala Gly
Lys Thr Gln Ile Phe Ile Pro Leu Lys Lys Asn Ile Leu Ser Gly
Asn Val Glu Ser Asp Ile Asp Ile Asp Ser Leu Val Thr Asp Lys
Val Val Ala Ala Leu Leu Ser Glu Asn Glu Ser Gly Val Asn Phe
Ala Arg Asp Ile Thr Asp Ile Gln Gly Glu Thr His Lys Ala Asp
Gln Asp Lys Ile Asp Ile Glu Leu Asp Asn Phe His Glu Ser Asp
Ser Asn Ile Thr Glu Thr Ile Glu Asn Leu Arg Asp Gln Leu Glu
Lys Ala Thr Asp Glu Glu His Lys Lys Glu Ile Glu Ser Gln Val
Asp Ala Lys Lys Lys Gln Lys Glu Glu Leu Asp Lys Lys Ala Ile
Asp Leu Asp Lys Ala Gln Gln Lys Leu Asp Phe Ala Glu Asp Asn
Leu Asp Ile Gln Arg Asp Thr Val Arg Glu Lys Leu Gln Glu Asn
Ile Asn Glu Thr Asn Lys Glu Lys Asn Leu Pro Lys Pro Gly Asp
Val Ser Ser Pro Lys Val Asp Lys Gln Leu Gln Ile Lys Glu Ser
Leu Glu Asp Leu Gln Glu Gln Leu Lys Glu Ala Ser Asp Glu Asn
Gln Lys Arg Glu Ile Glu Lys Gln Ile Glu Ile Lys Lys Asn Asp
Glu Glu Leu Phe Lys Asn Lys Asp His Lys Ala Leu Asp Leu Lys
Gln Glu Leu Asn Ser Lys Ala Ser Ser Lys Glu Lys Ile Glu Gly
Glu Glu Glu Asp Lys Glu Leu Asp Ser Lys Lys Asn Leu Glu Pro
Val Ser Glu Ala Asp Lys Val Asp Lys Ile Ser Lys Ser Asn Asn
Asn Glu Val Ser Lys Leu Ser Pro Leu Asp Glu Pro Ser Tyr Ser
Asp Ile Asp Ser Lys Glu Gly Val Asp Asn Lys Asp Val Asp Leu
Gln Lys Thr Lys Pro Gln Val Glu Ser Gln Pro Thr Ser Leu Asn
Glu Asp Leu Ile Asp Val Ser Ile Asp Ser Ser Asn Pro Val Phe
Leu Glu Val Ile Asp Pro Ile Thr Asn Leu Gly Thr Leu Gln Leu
Ile Asp Leu Asn Thr Gly Val Arg Leu Lys Glu Ser Ala Gln Gln
Gly Ile Gln Arg Tyr Gly Ile Tyr Glu Arg Glu Lys Asp Leu Val
Val Ile Lys Ile Asp Ser Gly Lys Ala Lys Leu Gln Ile Leu Asp
Lys Leu Glu Asn Leu Lys Val Ile Ser Glu Ser Asn Phe Glu Ile
Asn Lys Asn Ser Ser Leu Tyr Val Asp Ser Arg Met Ile Leu Val
Val Val Lys Asp Asp Ser Asn Ala Trp Arg Leu Ala Lys Phe Ser
Pro Lys Asn Leu Asp Glu Phe Ile Leu Ser Glu Asn Lys Ile Leu
Pro Phe Thr Ser Phe Ala Val Arg Lys Asn Phe Ile Tyr Leu Gln
Asp Glu Leu Lys Ser Leu Val Thr Leu Asp Val Asn Thr Leu Lys
Lys Val Lys
 oder eine Teilsequenz davon von wenigstens 10 Aminosäuren aufweist.

2. Immunologisch aktives Protein nach Anspruch 1, **dadurch gekennzeichnet, daß** es unter Verwendung von aus Borrelia burgdorfen isolierter DNA herstellbar ist.

3. Immunologisch aktives Protein nach Anspruch 2, **dadurch gekennzeichnet, daß** es unter Verwendung von aus Borrelia burgdorferi (DSM-Nr. 5662) isolierter DNA herstellbar ist.

4. Testkit zum Nachweis von Antikorpern gegen Borrelia-Stamme, **dadurch gekennzeichnet, daß** es wenigstens ein immunologisch aktives Protein nach einem der Ansprüche 1 bis 3 enthalt, das mit den in der Untersuchungsflüssigkeit vorhandenen Antikörpern reagieren kann und, daß es wenigstens eine Anzeigekomponente aufweist, die den Nachweis von Komplexen aus immunologisch aktivem Protein und Antikörper ermöglicht.

5. Testkit nach Anspruch 4, **dadurch gekennzeichnet, daß** es 2 bis 4 immunologisch aktive Proteine nach den Ansprüchen 1 bis 3 enthält.

6. Testkit nacn einem der Ansprüche 4 oder 5 , **dadurch gekennzeichnet, daß** die Anzeigekomponente ein gegen den nachzuweisenden Antikörper gerichteter Antikörper ist, der eine Markierung aufweist.

7. Testkit nach Anspruch 6 , **dadurch gekennzeichnet, daß** die Markierung in einem radioaktiven Isotop besteht.

8. Testkit nach einem der Ansprüche 4 bis 6 , **dadurch gekennzeichnet, daß** die Markierung aus einem Enzym besteht, das eine Farbreaktion katalysieren kann.

9. Testkit nach den Ansprüchen 4 oder 5, **dadurch gekennzeichnet, daß** das immunologisch aktive Protein oder ein dagegen gerichteter monoklonaler Antikörper biotinyliert ist und die Anzeigekomponente Avidin oder Streptavidin mit daran kovalent gebundenem Enzym, insbesondere Peroxydase ist.

10. Testkit nach einem der Ansprüche 4 bis 6 , **dadurch gekennzeichnet, daß** es ein ELISA-Testkit ist.

11. Testkit nach Anspruch 10 , **dadurch gekennzeichnet, daß** wenigstens ein immunologisch aktives Protein nach einem der Ansprüche 1 bis 3 an Mikrotiterplatten gekoppelt ist und die Anzeigekomponente aus Anti-human- Immunoglobulin, insbesondere IgG- und/oder IgM-Antikörpern besteht, an die ein eine Farbreaktion katalysierendes Enzym gekoppelt ist.

12. Verwendung von immunologisch aktiven Proteinen nach einem der Ansprüche 1 bis 3 zur Herstellung von Impfstoffen zum Schutz gegen Infektionen von Borrelia-Baklerien, vorzugsweise Borrelia burgdorferi-Stämmen.

## Patentansprüche (Patentansprüche für folgende(n) Vertragsstaat(en): ES)

1. Verfahren zur Herstellung eines immunologisch aktiven Proteins von Borrelia burgdorferi, das in einer von anderen aus Borrelia burgdorferi stammenden Proteinen freien Form vorliegt, und
a) die Aminosäuresequenz 
Met Lys Lys Asn Thr Leu Thr Ala Ile Leu Met Thr Leu Phe Leu
Phe Ile Ser Cys Asn Asn Ser Gly Lys Val Gly Ile Leu Thr Ser
Thr Asn Pro Ala Asp Glu Ser Ala Lys Gly Pro Asn Leu Thr Glu
Ile Ser Lys Lys Ile Thr Asp Ser Asn Ala Phe Val Leu Ala Val
Lys Glu Val Glu Thr Leu Val Leu Ser Ile Asp Glu Leu Ala Lys
Lys Ala Ile Gly Gln Lys Ile Asp Asn Asn Asn Gly Leu Ala Ala
Leu Asn Asn Gln Asn Gly Ser Leu Leu Ala Gly Ala Tyr Ala Ile
Ser Thr Leu Ile Thr Glu Lys Leu Ser Lys Leu Lys Asn Leu Glu
Glu Leu Lys Thr Glu Ile Ala Lys Ala Lys Lys Cys Ser Glu Glu
Phe Thr Asn Lys Leu Lys Ser Gly His Ala Asp Leu Gly Lys Gln
Asp Ala Thr Asp Asp His Ala Lys Ala Ala Ile Leu Lys Thr His
Ala Thr Thr Asp Lys Gly Ala Lys Glu Phe Lys Asp Leu Phe Glu
Ser Val Glu Gly Leu Leu Lys Ala Ala Gln Val Ala Leu Thr Asn
Ser Val Lys Glu Leu Thr Ser Pro Val Val Ala Glu Ser Pro Lys
Lys Pro
 oder eine Teilsequenz davon von wenigstens 10 Aminosäuren aufweist oder
b) die Aminosäuresequenz 
Met Lys Lys Asn Thr Leu Ser Ala Ile Leu Met Thr Leu Phe Leu
Phe Ile Ser Cys Asn Asn Ser Gly Lys Asp Gly Asn Thr Ser Ala
Asn Ser Ala Asp Glu Ser Val Lys Gly Pro Asn Leu Thr Glu Ile
Ser Lys Lys Ile Thr Asp Ser Asn Ala Val Leu Leu Ala Val Lys
Glu Val Glu Ala Leu Leu Ser Ser Ile Asp Glu Ile Ala Ala Lys
Ala Ile Gly Lys Lys Ile His Gln Asn Asn Gly Leu Asp Thr Glu
Asn Asn His Asn Gly Ser Leu Leu Ala Gly Ala Tyr Ala Ile Ser
Thr Leu Ile Lys Gln Lys Leu Asp Gly Leu Lys Asn Glu Gly Leu
Lys Glu Lys Ile Asp Ala Ala Lys Lys Cys Ser Glu Thr Phe Thr
Asn Lys Leu Lys Glu Lys His Thr Asp Leu Gly Lys Glu Gly Val
Thr Asp Ala Asp Ala Lys Glu Ala Ile Leu Lys Thr Asn Gly Thr
Lys Thr Lys Gly Ala Glu Glu Leu Gly Lys Leu Phe Glu Ser Val
Glu Val Leu Ser Lys Ala Ala Lys Glu Met Leu Ala Asn Ser Val
Lys Glu Leu Thr Ser Pro Val Val Ala Glu Ser Pro Lys Lys Pro
 oder eine Teilsequenz davon von wenigstens 10 Aminosäuren aufweist
oder
c) aus der Aminosäuresequenz 
Met Arg Gly Ser Ile Met Ile Ile Asn His Asn Thr Ser Ala Ile
Asn Ala Ser Arg Asn Asn Ala Ile Asn Ala Ala Asn Leu Ser Lys
Thr Gln Glu Lys Leu Ser Ser Asn Tyr Arg Ile Asn Arg Ala Ser
Asp Asp Ala Ala Gly Met Gly Val Ser Gly Lys Ile Asn Ala Gln
Ile Arg Gly Leu Ser Gln Ala Ser Arg Asn Thr Ser Lys Ala Ile
Asn Phe Ile Gln Thr Thr Glu Gly Asn Leu Asn Glu Val Glu Lys
Val Leu Val Arg Met Lys Glu Leu Ala Val Gln Ser Gly Asn Gly
Thr Tyr Ser Asp Ser Asp Arg Gly Ser Ile Gln Ile Glu Ile Glu
Gln Leu Thr Asp Glu Ile Asn Arg Ile Ala Asp Gln Ala Gln Tyr
Asn Gln Met His Met Leu Ser Asn Lys Ser Ala Ser Gln Asn Val
Lys Thr Ala Glu Glu Leu Gly Met Gln Pro Ala Lys Ile Asn Thr
Pro Ala Ser Leu Ser Gly Ser Gln Ala Ser Trp Thr Leu Arg Val
His Val Gly Ala Asn Gln Asp Glu Ala Ile Ala Val Asn Ile Tyr
Ser Ala Asn Val Ala Asn Leu Phe Ala Gly Glu Gly Ala Gln Ala
Ala Gln Ala Ala Pro Val Gln Glu Gly Ala Gln Glu Glu Gly Ala
Gln Gln Pro Thr Pro Ala Thr Ala Pro Thr Gln Gly Gly Val Asn
Ser Pro Val Asn Val Thr Thr Thr Val Asp Ala Asn Thr Ser Leu
Ala Lys Ile Glu Asn Ala Ile Arg Met Ile Ser Asp Gln Arg Ala
Asn Leu Gly Ala Phe Gln Asn Arg Leu Glu Ser Ile Lys Asn Ser
Thr Glu Tyr Ala Ile Glu Asn Leu Lys Ala Ser Tyr Ala Gln Ile
Lys Asp Ala Thr Met Thr Asp Glu Val Val Ala Ala Thr Thr Asn
Ser Ile Leu Thr Gln Ser Ala Met Ala Met Ile Ala Gln Ala Asn
Gln Val Pro Gln Tyr Val Leu Ser Leu Leu Arg
 oder der Teilsequenz dieses Proteins besteht, oder mit einer Deletion von 20-25 Aminosäuren am N-Terminus des Proteins
d) die Aminosäuresequenz 
Met Lys Lys Met Leu Leu Ile Phe Ser Phe Phe Leu Val Phe Leu
Asn Gly Phe Pro Leu Asn Ala Arg Glu Val Asp Lys Glu Lys Leu
Lys Asp Phe Val Asn Met Asp Leu Glu Phe Val Asn Tyr Lys Gly
Pro Tyr Asp Ser Thr Asn Thr Tyr Glu Gln Ile Val Gly Ile Gly
Glu Phe Leu Ala Arg Pro Leu Ile Asn Ser Asn Ser Asn Ser Ser
Tyr Tyr Gly Lys Tyr Phe Val Asn Arg Phe Ile Asp Asp Gln Asp
Lys Lys Ala Ser Val Asp Ile Phe Ser Ile Gly Ser Lys Ser Glu
Leu Asp Ser Ile Leu Asn Leu Arg Arg Ile Leu Thr Gly Tyr Leu
Ile Lys Ser Phe Asp Tyr Glu Arg Ser Ser Ala Glu Leu Ile Ala
Lys Ala Ile Thr Ile Tyr Asn Ala Val Tyr Arg Gly Asp Leu Asp
Tyr Tyr Lys Glu Phe Tyr Ile Glu Ala Ser Leu Lys Ser Leu Thr
Lys Glu Asn Ala Gly Leu Ser Arg Val Tyr Ser Gln Trp Ala Gly
Lys Thr Gln Ile Phe Ile Pro Leu Lys Lys Asn Ile Leu Ser Gly
Asn Val Glu Ser Asp Ile Asp Ile Asp Ser Leu Val Thr Asp Lys
Val Val Ala Ala Leu Leu Ser Glu Asn Glu Ser Gly Val Asn Phe
Ala Arg Asp Ile Thr Asp Ile Gln Gly Glu Thr His Lys Ala Asp
Gln Asp Lys Ile Asp Ile Glu Leu Asp Asn Phe His Glu Ser Asp
Ser Asn Ile Thr Glu Thr Ile Glu Asn Leu Arg Asp Gln Leu Glu
Lys Ala Thr Asp Glu Glu His Lys Lys Glu Ile Glu Ser Gln Val
Asp Ala Lys Lys Lys Gln Lys Glu Glu Leu Asp Lys Lys Ala Ile
Asp Leu Asp Lys Ala Gln Gln Lys Leu Asp Phe Ala Glu Asp Asn
Leu Asp Ile Gln Arg Asp Thr Val Arg Glu Lys Leu Gln Glu Asn
Ile Asn Glu Thr Asn Lys Glu Lys Asn Leu Pro Lys Pro Gly Asp
Val Ser Ser Pro Lys Val Asp Lys Gln Leu Gln Ile Lys Glu Ser
Leu Glu Asp Leu Gln Glu Gln Leu Lys Glu Ala Ser Asp Glu Asn
Gln Lys Arg Glu Ile Glu Lys Gln Ile Glu Ile Lys Lys Asn Asp
Glu Glu Leu Phe Lys Asn Lys Asp His Lys Ala Leu Asp Leu Lys
Gln Glu Leu Asn Ser Lys Ala Ser Ser Lys Glu Lys Ile Glu Gly
Glu Glu Glu Asp Lys Glu Leu Asp Ser Lys Lys Asn Leu Glu Pro
Val Ser Glu Ala Asp Lys Val Asp Lys Ile Ser Lys Ser Asn Asn
Asn Glu Val Ser Lys Leu Ser Pro Leu Asp Glu Pro Ser Tyr Ser
Asp Ile Asp Ser Lys Glu Gly Val Asp Asn Lys Asp Val Asp Leu
Gln Lys Thr Lys Pro Gln Val Glu Ser Gln Pro Thr Ser Leu Asn
Glu Asp Leu Ile Asp Val Ser Ile Asp Ser Ser Asn Pro Val Phe
Leu Glu Val Ile Asp Pro Ile Thr Asn Leu Gly Thr Leu Gln Leu
Ile Asp Leu Asn Thr Gly Val Arg Leu Lys Glu Ser Ala Gln Gln
Gly Ile Gln Arg Tyr Gly Ile Tyr Glu Arg Glu Lys Asp Leu Val
Val Ile Lys Ile Asp Ser Gly Lys Ala Lys Leu Gln Ile Leu Asp
Lys Leu Glu Asn Leu Lys Val Ile Ser Glu Ser Asn Phe Glu Ile
Asn Lys Asn Ser Ser Leu Tyr Val Asp Ser Arg Met Ile Leu Val
Val Val Lys Asp Asp Ser Asn Ala Trp Arg Leu Ala Lys Phe Ser
Pro Lys Asn Leu Asp Glu Phe Ile Leu Ser Glu Asn Lys Ile Leu
Pro Phe Thr Ser Phe Ala Val Arg Lys Asn Phe Ile Tyr Leu Gln
Asp Glu Leu Lys Ser Leu Val Thr Leu Asp Val Asn Thr Leu Lys
Lys Val Lys
 oder eine Teilsequenz davon von wenigstens 10 Aminosäuren aufweist, **dadurch gekennzeichnet, daß** es gentechnologisch hergestellt wird.

2. Verfahren nach Anspruch 1, **dadurch gekennzeichnet, daß** das immunologisch aktive Protein unter Verwendung von aus Borrelia burgdorferi isolierter DNA herstellbar ist.

3. Verfahren nach Anspruch 2, **dadurch gekennzeichnet, daß** das immunologisch aktive Protein unter Verwendung von aus Borrelia burgdorferi (DSM-Nr. 5662) isolierter DNA herstellbar ist.

4. Testkit zum Nachweis von Antikörpern gegen Borrelia-Stämme, **dadurch gekennzeichnet, daß** es wenigstens ein immunologisch aktives Protein enthält, das nach einem der Ansprüche 1 bis 3 herstellbar ist und das mit den in der Untersuchungsflüssigkeit vorhandenen Antikörpern reagieren kann und, daß es wenigstens eine Anzeigekomponente aufweist, die den Nachweis von Komplexen aus immunologisch aktivem Protein und Antikörper ermöglicht.

5. Testkit nach Anspruch 4, **dadurch gekennzeichnet, daß** es 2 bis 4 immunologisch aktive Proteine, die nach den Ansprüchen 1 bis 3 herstellbar sind, enthält.

6. Testkit nach einem der Ansprüche 4 oder 5 , **dadurch gekennzeichnet daß** die Anzeigekomponente ein gegen den nachzuweisenden Antikörper gerichteter Antikörper ist, der eine Markierung aufweist.

7. Testkit nach Anspruch 6 , **dadurch gekennzeichnet, daß** die Markierung in einem radioaktiven Isotop besteht.

8. Testkit nach einem der Ansprüche 4 bis 6 , **dadurch gekennzeichnet, daß** die Markierung aus einem Enzym besteht, das eine Farbreaktion katalysieren kann.

9. Testkit nach den Ansprüchen 4 oder 5 , **dadurch gekennzeichnet, daß** das immunologisch aktive Protein oder ein dagegen gerichteter monoklonaler Antikörper biotinyliert ist und die Anzeigekomponente Avidin oder Streptavidin mit daran kovalent gebundenem Enzym, insbesondere Peroxydase ist.

10. Testkit nach einem der Ansprüche 4 bis 6, **dadurch gekennzeichnet, daß** es ein ELISA-Testkit ist.

11. Testkit nach Anspruch 10, **dadurch gekennzeichnet, daß** wenigstens ein immunologisch aktives Protein nach einem der Ansprüche 1 bis 3 an Mikrotiterplatten gekoppelt ist und die Anzeigekomponente aus Anti-human- Immunoglobulin. insbesondere IgG- und/oder IgM-Antikörpern besteht, an die ein eine Farbreaktion katalysierendes Enzym gekoppelt ist.

12. Verfahren zur Herstellung von Impfstoffen zum Schutz gegen Infektionen von Borrelia-Bakterien, vorzugsweise Borrelia burgdorferi-Stämmen, **dadurch gekennzeichnet, daß** immunologisch aktive Proteine, die nach einem der Ansprüche 1 bis 3 herstellbar sind, verwendet werden.

## Claims (Claims for the following Contracting State(s): AT, BE, CH, DE, DK, FR, GB, IT, LI, LU NL, SE)

1. An immunologically active protein from Borrelia burgdorferi, which is in a form free of other proteins originating from Borrelia burgdorferi and has been prepared by genetic manipulation, **characterized in that** it
a) has the amino acid sequence 
Met Lys Lys Asn Thr Leu Thr Ala Ile Leu Met Thr Leu Phe Leu
Phe Ile Ser Cys Asn Asn Ser Gly Lys Val Gly Ile Leu Thr Ser
Thr Asn Pro Ala Asp Glu Ser Ala Lys Gly Pro Asn Leu Thr Glu
Ile Ser Lys Lys Ile Thr Asp Ser Asn Ala Phe Val Leu Ala Val
Lys Glu Val Glu Thr Leu Val Leu Ser Ile Asp Glu Leu Ala Lys
Lys Ala Ile Gly Gln Lys Ile Asp Asn Asn Asn Gly Leu Ala Ala
Leu Asn Asn Gln Asn Gly Ser Leu Leu Ala Gly Ala Tyr Ala Ile
Ser Thr Leu Ile Thr Glu Lys Leu Ser Lys Leu Lys Asn Leu Glu
Glu Leu Lys Thr Glu Ile Ala Lys Ala Lys Lys Cys Ser Glu Glu
Phe Thr Asn Lys Leu Lys Ser Gly His Ala Asp Leu Gly Lys Gln
Asp Ala Thr Asp Asp His Ala Lys Ala Ala Ile Leu Lys Thr His
Ala Thr Thr Asp Lys Gly Ala Lys Glu Phe Lys Asp Leu Phe Glu
Ser Val Glu Gly Leu Leu Lys Ala Ala Gln Val Ala Leu Thr Asn
Ser Val Lys Glu Leu Thr Ser Pro Val Val Ala Glu Ser Pro Lys
Lys Pro
 or a partial sequence thereof having at least 10 amino acids or
b) has the amino acid sequence 
Met Lys Lys Asn Thr Leu Ser Ala Ile Leu Met Thr Leu Phe Leu
Phe Ile Ser Cys Asn Asn Ser Gly Lys Asp Gly Asn Thr Ser Ala
Asn Ser Ala Asp Glu Ser Val Lys Gly Pro Asn Leu Thr Glu Ile
Ser Lys Lys Ile Thr Asp Ser Asn Ala Val Leu Leu Ala Val Lys
Glu Val Glu Ala Leu Leu Ser Ser Ile Asp Glu Ile Ala Ala Lys
Ala Ile Gly Lys Lys Ile His Gln Asn Asn Gly Leu Asp Thr Glu
Asn Asn His Asn Gly Ser Leu Leu Ala Gly Ala Tyr Ala Ile Ser
Thr Leu Ile Lys Gln Lys Leu Asp Gly Leu Lys Asn Glu Gly Leu
Lys Glu Lys Ile Asp Ala Ala Lys Lys Cys Ser Glu Thr Phe Thr
Asn Lys Leu Lys Glu Lys His Thr Asp Leu Gly Lys Glu Gly Val
Thr Asp Ala Asp Ala Lys Glu Ala Ile Leu Lys Thr Asn Gly Thr
Lys Thr Lys Gly Ala Glu Glu Leu Gly Lys Leu Phe Glu Ser Val
Glu Val Leu Ser Lys Ala Ala Lys Glu Met Leu Ala Asn Ser Val
Lys Glu Leu Thr Ser Pro Val Val Ala Glu Ser Pro Lys Lys Pro
 or a partial sequence thereof having at least 10 amino acids or
c) consists of the amino acid sequence 
Met Arg Gly Ser Ile Met Ile Ile Asn His Asn Thr Ser Ala Ile
Asn Ala Ser Arg Asn Asn Ala Ile Asn Ala Ala Asn Leu Ser Lys
Thr Gln Glu Lys Leu Ser Ser Asn Tyr Arg Ile Asn Arg Ala Ser
Asp Asp Ala Ala Gly Met Gly Val Ser Gly Lys Ile Asn Ala Gln
Ile Arg Gly Leu Ser Gln Ala Ser Arg Asn Thr Ser Lys Ala Ile
Asn Phe Ile Gln Thr Thr Glu Gly Asn Leu Asn Glu Val Glu Lys
Val Leu Val Arg Met Lys Glu Leu Ala Val Gln Ser Gly Asn Gly
Thr Tyr Ser Asp Ser Asp Arg Gly Ser Ile Gln Ile Glu Ile Glu
Gln Leu Thr Asp Glu Ile Asn Arg Ile Ala Asp Gln Ala Gln Tyr
Asn Gln Met His Met Leu Ser Asn Lys Ser Ala Ser Gln Asn Val
Lys Thr Ala Glu Glu Leu Gly Met Gln Pro Ala Lys Ile Asn Thr
Pro Ala Ser Leu Ser Gly Ser Gln Ala Ser Trp Thr Leu Arg Val
His Val Gly Ala Asn Gln Asp Glu Ala Ile Ala Val Asn Ile Tyr
Ser Ala Asn Val Ala Asn Leu Phe Ala Gly Glu Gly Ala Gln Ala
Ala Gln Ala Ala Pro Val Gln Glu Gly Ala Gln Glu Glu Gly Ala
Gln Gln Pro Thr Pro Ala Thr Ala Pro Thr Gln Gly Gly Val Asn
Ser Pro Val Asn Val Thr Thr Thr Val Asp Ala Asn Thr Ser Leu
Ala Lys Ile Glu Asn Ala Ile Arg Met Ile Ser Asp Gln Arg Ala
Asn Leu Gly Ala Phe Gln Asn Arg Leu Glu Ser Ile Lys Asn Ser
Thr Glu Tyr Ala Ile Glu Asn Leu Lys Ala Ser Tyr Ala Gln Ile
Lys Asp Ala Thr Met Thr Asp Glu Val Val Ala Ala Thr Thr Asn
Ser Ile Leu Thr Gln Ser Ala Met Ala Met Ile Ala Gln Ala Asn
Gln Val Pro Gln Tyr Val Leu Ser Leu Leu Arg
 or the partial sequence of this protein with a deletion of 20-25 amino acids at the N-terminus of the protein or
d) has the amino acid sequence 
Met Lys Lys Met Leu Leu Ile Phe Ser Phe Phe Leu Val Phe Leu
Asn Gly Phe Pro Leu Asn Ala Arg Glu Val Asp Lys Glu Lys Leu
Lys Asp Phe Val Asn Met Asp Leu Glu Phe Val Asn Tyr Lys Gly
Pro Tyr Asp Ser Thr Asn Thr Tyr Glu Gln Ile Val Gly Ile Gly
Glu Phe Leu Ala Arg Pro Leu Ile Asn Ser Asn Ser Asn Ser Ser
Tyr Tyr Gly Lys Tyr Phe Val Asn Arg Phe Ile Asp Asp Gln Asp
Lys Lys Ala Ser Val Asp Ile Phe Ser Ile Gly Ser Lys Ser Glu
Leu Asp Ser Ile Leu Asn Leu Arg Arg Ile Leu Thr Gly Tyr Leu
Ile Lys Ser Phe Asp Tyr Glu Arg Ser Ser Ala Glu Leu Ile Ala
Lys Ala Ile Thr Ile Tyr Asn Ala Val Tyr Arg Gly Asp Leu Asp
Tyr Tyr Lys Glu Phe Tyr Ile Glu Ala Ser Leu Lys Ser Leu Thr
Lys Glu Asn Ala Gly Leu Ser Arg Val Tyr Ser Gln Trp Ala Gly
Lys Thr Gln Ile Phe Ile Pro Leu Lys Lys Asn Ile Leu Ser Gly
Asn Val Glu Ser Asp Ile Asp Ile Asp Ser Leu Val Thr Asp Lys
Val Val Ala Ala Leu Leu Ser Glu Asn Glu Ser Gly Val Asn Phe
Ala Arg Asp Ile Thr Asp Ile Gln Gly Glu Thr His Lys Ala Asp
Gln Asp Lys Ile Asp Ile Glu Leu Asp Asn Phe His Glu Ser Asp
Ser Asn Ile Thr Glu Thr Ile Glu Asn Leu Arg Asp Gln Leu Glu
Lys Ala Thr Asp Glu Glu His Lys Lys Glu Ile Glu Ser Gln Val
Asp Ala Lys Lys Lys Gln Lys Glu Glu Leu Asp Lys Lys Ala Ile
Asp Leu Asp Lys Ala Gln Gln Lys Leu Asp Phe Ala Glu Asp Asn
Leu Asp Ile Gln Arg Asp Thr Val Arg Glu Lys Leu Gln Glu Asn
Ile Asn Glu Thr Asn Lys Glu Lys Asn Leu Pro Lys Pro Gly Asp
Val Ser Ser Pro Lys Val Asp Lys Gln Leu Gln Ile Lys Glu Ser
Leu Glu Asp Leu Gln Glu Gln Leu Lys Glu Ala Ser Asp Glu Asn
Gln Lys Arg Glu Ile Glu Lys Gln Ile Glu Ile Lys Lys Asn Asp
Glu Glu Leu Phe Lys Asn Lys Asp His Lys Ala Leu Asp Leu Lys
Gln Glu Leu Asn Ser Lys Ala Ser Ser Lys Glu Lys Ile Glu Gly
Glu Glu Glu Asp Lys Glu Leu Asp Ser Lys Lys Asn Leu Glu Pro
Val Ser Glu Ala Asp Lys Val Asp Lys Ile Ser Lys Ser Asn Asn
Asn Glu Val Ser Lys Leu Ser Pro Leu Asp Glu Pro Ser Tyr Ser
Asp Ile Asp Ser Lys Glu Gly Val Asp Asn Lys Asp Val Asp Leu
Gln Lys Thr Lys Pro Gln Val Glu Ser Gln Pro Thr Ser Leu Asn
Glu Asp Leu Ile Asp Val Ser Ile Asp Ser Ser Asn Pro Val Phe
Leu Glu Val Ile Asp Pro Ile Thr Asn Leu Gly Thr Leu Gln Leu
Ile Asp Leu Asn Thr Gly Val Arg Leu Lys Glu Ser Ala Gln Gln
Gly Ile Gln Arg Tyr Gly Ile Tyr Glu Arg Glu Lys Asp Leu Val
Val Ile Lys Ile Asp Ser Gly Lys Ala Lys Leu Gln Ile Leu Asp
Lys Leu Glu Asn Leu Lys Val Ile Ser Glu Ser Asn Phe Glu Ile
Asn Lys Asn Ser Ser Leu Tyr Val Asp Ser Arg Met Ile Leu Val
Val Val Lys Asp Asp Ser Asn Ala Trp Arg Leu Ala Lys Phe Ser
Pro Lys Asn Leu Asp Glu Phe Ile Leu Ser Glu Asn Lys Ile Leu
Pro Phe Thr Ser Phe Ala Val Arg Lys Asn Phe Ile Tyr Leu Gln
Asp Glu Leu Lys Ser Leu Val Thr Leu Asp Val Asn Thr Leu Lys
Lys Val Lys
 or a partial sequence thereof having at least 10 amino acids.

2. The immunologically active protein as claimed in claim 1, **characterized in that** it can be prepared using DNA isolated from Borrelia burgdorferi.

3. The immunologically active protein as claimed in claim 2, **characterized in that** it can be prepared using DNA isolated from Borrelia burgdorferi (DSM No. 5662).

4. An assay kit for detecting antibodies against Borrelia strains, **characterized in that** it contains at least one immunologically active protein as claimed in one of claims 1 to 3, which protein is able to react with the antibodies present in the test fluid, and which kit has at least one indicator component which makes it possible to detect complexes of immunologically active protein and antibody.

5. The assay kit as claimed in claim 4, **characterized in that** it contains 2 to 4 immunologically active proteins as claimed in claims 1 to 3.

6. The assay kit as claimed in any of claims 4 or 5, **characterized in that** the indicator component is an antibody which is directed against the antibody to be detected and which has a label.

7. The assay kit as claimed in claim 6, **characterized in that** the label is a radioactive isotope.

8. The assay kit as claimed in any of claims 4 to 6, **characterized in that** the label consists of an enzyme which is able to catalyze a color reaction.

9. The assay kit as claimed in claim 4 or 5, **characterized in that** the immunologically active protein or a monoclonal antibody directed against it is biotinylated, and the indicator component is avidin or streptavidin with an enzyme covalently bonded thereto, in particular peroxidase.

10. The assay kit as claimed in any of claims 4 to 6, **characterized in that** it is an ELISA assay kit.

11. The assay kit as claimed in claim 10, **characterized in that** at least one immunologically active protein as claimed in any of claims 1 to 3 is coupled to microtiter plates, and the indicator component consists of anti-human immunoglobulin, in particular IgG and/or IgM antibodies to which an enzyme catalyzing a color reaction is coupled.

12. The use of immunologically active proteins as claimed in any of claims 1 to 3 for preparing vaccines for protection against infection by Borrelia bacteria, preferably Borrelia burgdorferi strains.

## Claims (Claims for the following Contracting State(s): ES)

1. A process for the production of an immunologically active protein from Borrelia burgdorferi, which is in a form free of other proteins originating from Borrelia burgdorferi and
a) has the amino acid sequence 
Met Lys Lys Asn Thr Leu Thr Ala Ile Leu Met Thr Leu Phe Leu
Phe Ile Ser Cys Asn Asn Ser Gly Lys Val Gly Ile Leu Thr Ser
Thr Asn Pro Ala Asp Glu Ser Ala Lys Gly Pro Asn Leu Thr Glu
Ile Ser Lys Lys Ile Thr Asp Ser Asn Ala Phe Val Leu Ala Val
Lys Glu Val Glu Thr Leu Val Leu Ser Ile Asp Glu Leu Ala Lys
Lys Ala Ile Gly Gln Lys Ile Asp Asn Asn Asn Gly Leu Ala Ala
Leu Asn Asn Gln Asn Gly Ser Leu Leu Ala Gly Ala Tyr Ala Ile
Ser Thr Leu Ile Thr Glu Lys Leu Ser Lys Leu Lys Asn Leu Glu
Glu Leu Lys Thr Glu Ile Ala Lys Ala Lys Lys Cys Ser Glu Glu
Phe Thr Asn Lys Leu Lys Ser Gly His Ala Asp Leu Gly Lys Gln
Asp Ala Thr Asp Asp His Ala Lys Ala Ala Ile Leu Lys Thr His
Ala Thr Thr Asp Lys Gly Ala Lys Glu Phe Lys Asp Leu Phe Glu
Ser Val Glu Gly Leu Leu Lys Ala Ala Gln Val Ala Leu Thr Asn
Ser Val Lys Glu Leu Thr Ser Pro Val Val Ala Glu Ser Pro Lys
Lys Pro
 or a partial sequence thereof having at least 10 amino acids or
b) has the amino acid sequence 
Met Lys Lys Asn Thr Leu Ser Ala Ile Leu Met Thr Leu Phe Leu
Phe Ile Ser Cys Asn Asn Ser Gly Lys Asp Gly Asn Thr Ser Ala
Asn Ser Ala Asp Glu Ser Val Lys Gly Pro Asn Leu Thr Glu Ile
Ser Lys Lys Ile Thr Asp Ser Asn Ala Val Leu Leu Ala Val Lys
Glu Val Glu Ala Leu Leu Ser Ser Ile Asp Glu Ile Ala Ala Lys
Ala Ile Gly Lys Lys Ile His Gln Asn Asn Gly Leu Asp Thr Glu
Asn Asn His Asn Gly Ser Leu Leu Ala Gly Ala Tyr Ala Ile Ser
Thr Leu Ile Lys Gln Lys Leu Asp Gly Leu Lys Asn Glu Gly Leu
Lys Glu Lys Ile Asp Ala Ala Lys Lys Cys Ser Glu Thr Phe Thr
Asn Lys Leu Lys Glu Lys His Thr Asp Leu Gly Lys Glu Gly Val
Thr Asp Ala Asp Ala Lys Glu Ala Ile Leu Lys Thr Asn Gly Thr
Lys Thr Lys Gly Ala Glu Glu Leu Gly Lys Leu Phe Glu Ser Val
Glu Val Leu Ser Lys Ala Ala Lys Glu Met Leu Ala Asn Ser Val
Lys Glu Leu Thr Ser Pro Val Val Ala Glu Ser Pro Lys Lys Pro
 or a partial sequence thereof having at least 10 amino acids or
c) consists of the amino acid sequence 
Met Arg Gly Ser Ile Met Ile Ile Asn His Asn Thr Ser Ala Ile
Asn Ala Ser Arg Asn Asn Ala Ile Asn Ala Ala Asn Leu Ser Lys
Thr Gln Glu Lys Leu Ser Ser Asn Tyr Arg Ile Asn Arg Ala Ser
Asp Asp Ala Ala Gly Met Gly Val Ser Gly Lys Ile Asn Ala Gln
Ile Arg Gly Leu Ser Gln Ala Ser Arg Asn Thr Ser Lys Ala Ile
Asn Phe Ile Gln Thr Thr Glu Gly Asn Leu Asn Glu Val Glu Lys
Val Leu Val Arg Met Lys Glu Leu Ala Val Gln Ser Gly Asn Gly
Thr Tyr Ser Asp Ser Asp Arg Gly Ser Ile Gln Ile Glu Ile Glu
Gln Leu Thr Asp Glu Ile Asn Arg Ile Ala Asp Gln Ala Gln Tyr
Asn Gln Met His Met Leu Ser Asn Lys Ser Ala Ser Gln Asn Val
Lys Thr Ala Glu Glu Leu Gly Met Gln Pro Ala Lys Ile Asn Thr
Pro Ala Ser Leu Ser Gly Ser Gln Ala Ser Trp Thr Leu Arg Val
His Val Gly Ala Asn Gln Asp Glu Ala Ile Ala Val Asn Ile Tyr
Ser Ala Asn Val Ala Asn Leu Phe Ala Gly Glu Gly Ala Gln Ala
Ala Gln Ala Ala Pro Val Gln Glu Gly Ala Gln Glu Glu Gly Ala
Gln Gln Pro Thr Pro Ala Thr Ala Pro Thr Gln Gly Gly Val Asn
Ser Pro Val Asn Val Thr Thr Thr Val Asp Ala Asn Thr Ser Leu
Ala Lys Ile Glu Asn Ala Ile Arg Met Ile Ser Asp Gln Arg Ala
Asn Leu Gly Ala Phe Gln Asn Arg Leu Glu Ser Ile Lys Asn Ser
Thr Glu Tyr Ala Ile Glu Asn Leu Lys Ala Ser Tyr Ala Gln Ile
Lys Asp Ala Thr Met Thr Asp Glu Val Val Ala Ala Thr Thr Asn
Ser Ile Leu Thr Gln Ser Ala Met Ala Met Ile Ala Gln Ala Asn
Gln Val Pro Gln Tyr Val Leu Ser Leu Leu Arg
 or the partial sequence of this protein with a deletion of 20-25 amino acids at the N-terminus of the protein or
d) has the amino acid sequence 
Met Lys Lys Met Leu Leu Ile Phe Ser Phe Phe Leu Val Phe Leu
Asn Gly Phe Pro Leu Asn Ala Arg Glu Val Asp Lys Glu Lys Leu
Lys Asp Phe Val Asn Met Asp Leu Glu Phe Val Asn Tyr Lys Gly
Pro Tyr Asp Ser Thr Asn Thr Tyr Glu Gln Ile Val Gly Ile Gly
Glu Phe Leu Ala Arg Pro Leu Ile Asn Ser Asn Ser Asn Ser Ser
Tyr Tyr Gly Lys Tyr Phe Val Asn Arg Phe Ile Asp Asp Gln Asp
Lys Lys Ala Ser Val Asp Ile Phe Ser Ile Gly Ser Lys Ser Glu
Leu Asp Ser Ile Leu Asn Leu Arg Arg Ile Leu Thr Gly Tyr Leu
Ile Lys Ser Phe Asp Tyr Glu Arg Ser Ser Ala Glu Leu Ile Ala
Lys Ala Ile Thr Ile Tyr Asn Ala Val Tyr Arg Gly Asp Leu Asp
Tyr Tyr Lys Glu Phe Tyr Ile Glu Ala Ser Leu Lys Ser Leu Thr
Lys Glu Asn Ala Gly Leu Ser Arg Val Tyr Ser Gln Trp Ala Gly
Lys Thr Gln Ile Phe Ile Pro Leu Lys Lys Asn Ile Leu Ser Gly
Asn Val Glu Ser Asp Ile Asp Ile Asp Ser Leu Val Thr Asp Lys
Val Val Ala Ala Leu Leu Ser Glu Asn Glu Ser Gly Val Asn Phe
Ala Arg Asp Ile Thr Asp Ile Gln Gly Glu Thr His Lys Ala Asp
Gln Asp Lys Ile Asp Ile Glu Leu Asp Asn Phe His Glu Ser Asp
Ser Asn Ile Thr Glu Thr Ile Glu Asn Leu Arg Asp Gln Leu Glu
Lys Ala Thr Asp Glu Glu His Lys Lys Glu Ile Glu Ser Gln Val
Asp Ala Lys Lys Lys Gln Lys Glu Glu Leu Asp Lys Lys Ala Ile
Asp Leu Asp Lys Ala Gln Gln Lys Leu Asp Phe Ala Glu Asp Asn
Leu Asp Ile Gln Arg Asp Thr Val Arg Glu Lys Leu Gln Glu Asn
Ile Asn Glu Thr Asn Lys Glu Lys Asn Leu Pro Lys Pro Gly Asp
Val Ser Ser Pro Lys Val Asp Lys Gln Leu Gln Ile Lys Glu Ser
Leu Glu Asp Leu Gln Glu Gln Leu Lys Glu Ala Ser Asp Glu Asn
Gln Lys Arg Glu Ile Glu Lys Gln Ile Glu Ile Lys Lys Asn Asp
Glu Glu Leu Phe Lys Asn Lys Asp His Lys Ala Leu Asp Leu Lys
Gln Glu Leu Asn Ser Lys Ala Ser Ser Lys Glu Lys Ile Glu Gly
Glu Glu Glu Asp Lys Glu Leu Asp Ser Lys Lys Asn Leu Glu Pro
Val Ser Glu Ala Asp Lys Val Asp Lys Ile Ser Lys Ser Asn Asn
Asn Glu Val Ser Lys Leu Ser Pro Leu Asp Glu Pro Ser Tyr Ser
Asp Ile Asp Ser Lys Glu Gly Val Asp Asn Lys Asp Val Asp Leu
Gln Lys Thr Lys Pro Gln Val Glu Ser Gln Pro Thr Ser Leu Asn
Glu Asp Leu Ile Asp Val Ser Ile Asp Ser Ser Asn Pro Val Phe
Leu Glu Val Ile Asp Pro Ile Thr Asn Leu Gly Thr Leu Gln Leu
Ile Asp Leu Asn Thr Gly Val Arg Leu Lys Glu Ser Ala Gln Gln
Gly Ile Gln Arg Tyr Gly Ile Tyr Glu Arg Glu Lys Asp Leu Val
Val Ile Lys Ile Asp Ser Gly Lys Ala Lys Leu Gln Ile Leu Asp
Lys Leu Glu Asn Leu Lys Val Ile Ser Glu Ser Asn Phe Glu Ile
Asn Lys Asn Ser Ser Leu Tyr Val Asp Ser Arg Met Ile Leu Val
Val Val Lys Asp Asp Ser Asn Ala Trp Arg Leu Ala Lys Phe Ser
Pro Lys Asn Leu Asp Glu Phe Ile Leu Ser Glu Asn Lys Ile Leu
Pro Phe Thr Ser Phe Ala Val Arg Lys Asn Phe Ile Tyr Leu Gln
Asp Glu Leu Lys Ser Leu Val Thr Leu Asp Val Asn Thr Leu Lys
Lys Val Lys
 or a partial sequence thereof having at least 10 amino acids, **characterized in that** it is prepared by genetic manipulation.

2. The process as claimed in claim 1, **characterized in that** the immunologically active protein can be prepared using DNA isolated from Borrelia burgdorferi.

3. The process as claimed in claim 2, **characterized in that** the immunologically active protein can be prepared using DNA isolated from Borrelia burgdorferi (DSM No. 5662).

4. An assay kit for detecting antibodies against Borrelia strains, **characterized in that** it contains at least one immunologically active protein which can be prepared as claimed in one of claims 1 to 3, which protein is able to react with the antibodies present in the test fluid, and which kit has at least one indicator component which makes it possible to detect complexes of immunologically active protein and antibody.

5. The assay kit as claimed in claim 4, **characterized in that** it contains 2 to 4 immunologically active proteins which can be prepared as claimed in claims 1 to 3.

6. The assay kit as claimed in any of claims 4 or 5, **characterized in that** the indicator component is an antibody which is directed against the antibody to be detected and which has a label.

7. The assay kit as claimed in claim 6, **characterized in that** the label is a radioactive isotope.

8. The assay kit as claimed in any of claims 4 to 6, **characterized in that** the label consists of an enzyme which is able to catalyze a color reaction.

9. The assay kit as claimed in claim 4 or 5, **characterized in that** the immunologically active protein or a monoclonal antibody directed against it is biotinylated, and the indicator component is avidin or streptavidin with an enzyme covalently bonded thereto, in particular peroxidase.

10. The assay kit as claimed in any of claims 4 to 6, **characterized in that** it is an ELISA assay kit.

11. The assay kit as claimed in claim 10, **characterized in that** at least one immunologically active protein as claimed in any of claims 1 to 3 is coupled to microtiter plates, and the indicator component consists of anti-human immunoglobulin, in particular IgG and/or IgM antibodies to which an enzyme catalyzing a color reaction is coupled.

12. A process for preparing vaccines for protection against infection by Borrelia bacteria, preferably Borrelia burgdorferi strains, **characterized in that** immunologically active proteins which can be prepared as claimed in any of claims 1 to 3 are used.

## Revendications (Revendications pour l'(les) Etat(s) contractant(s) suivant(s): AT, BE, CH, DE, DK, FR, GB, IT, LI, LU, NL, SE)

1. Protéine immunologiquement active de Borrelia burgdorferi qui se présente sous une forme dépourvue d'autres protéines issues de Borrelia burgdorferi et qui a été préparée par la technique génétique, **caractérisée en ce que**
a) elle présente la séquence d'aminoacides 
Met Lys Lys Asn Thr Leu Thr Ala Ile Leu Met Thr Leu Phe Leu
Phe Ile Ser Cys Asn Asn Ser Gly Lys Val Gly Ile Leu Thr Ser
Thr Asn Pro Ala Asp Glu Ser Ala Lys Gly Pro Asn Leu Thr Glu
Ile Ser Lys Lys Ile Thr Asp Ser Asn Ala Phe Val Leu Ala Val
Lys Glu Val Glu Thr Leu Val Leu Ser Ile Asp Glu Leu Ala Lys
Lys Ala Ile Gly Gln Lys Ile Asp Asn Asn Asn Gly Leu Ala Ala
Leu Asn Asn Gln Asn Gly Ser Leu Leu Ala Gly Ala Tyr Ala Ile
Ser Thr Leu Ile Thr Glu Lys Leu Ser Lys Leu Lys Asn Leu Glu
Glu Leu Lys Thr Glu Ile Ala Lys Ala Lys Lys Cys Ser Glu Glu
Phe Thr Asn Lys Leu Lys Ser Gly His Ala Asp Leu Gly Lys Gln
Asp Ala Thr Asp Asp His Ala Lys Ala Ala Ile Leu Lys Thr His
Ala Thr Thr Asp Lys Gly Ala Lys Glu Phe Lys Asp Leu Phe Glu
Ser Val Glu Gly Leu Leu Lys Ala Ala Gln Val Ala Leu Thr Asn
Ser Val Lys Glu Leu Thr Ser Pro Val Val Ala Glu Ser Pro Lys
Lys Pro
 ou une séquence partielle de celle-ci d'au moins 10 aminoacides
ou
b) elle présente la séquence d'aminoacides 
Met Lys Lys Asn Thr Leu Ser Ala Ile Leu Met Thr Leu Phe Leu
Phe Ile Ser Cys Asn Asn Ser Gly Lys Asp Gly Asn Thr Ser Ala
Asn Ser Ala Asp Glu Ser Val Lys Gly Pro Asn Leu Thr Glu Ile
Ser Lys Lys Ile Thr Asp Ser Asn Ala Val Leu Leu Ala Val Lys
Glu Val Glu Ala Leu Leu Ser Ser Ile Asp Glu Ile Ala Ala Lys
Ala Ile Gly Lys Lys Ile His Gln Asn Asn Gly Leu Asp Thr Glu
Asn Asn His Asn Gly Ser Leu Leu Ala Gly Ala Tyr Ala Ile Ser
Thr Leu Ile Lys Gln Lys Leu Asp Gly Leu Lys Asn Glu Gly Leu
Lys Glu Lys Ile Asp Ala Ala Lys Lys Cys Ser Glu Thr Phe Thr
Asn Lys Leu Lys Glu Lys His Thr Asp Leu Gly Lys Glu Gly Val
Thr Asp Ala Asp Ala Lys Glu Ala Ile Leu Lys Thr Asn Gly Thr
Lys Thr Lys Gly Ala Glu Glu Leu Gly Lys Leu Phe Glu Ser Val
Glu Val Leu Ser Lys Ala Ala Lys Glu Met Leu Ala Asn Ser Val
Lys Glu Leu Thr Ser Pro Val Val Ala Glu Ser Pro Lys Lys Pro
 ou une séquence partielle de celle-ci d'au moins 10 aminoacides
ou
c) elle est constituée de la séquence d'aminoacides 
Met Arg Gly Ser Ile Met Ile Ile Asn His Asn Thr Ser Ala Ile
Asn Ala Ser Arg Asn Asn Ala Ile Asn Ala Ala Asn Leu Ser Lys
Thr Gln Glu Lys Leu Ser Ser Asn Tyr Arg Ile Asn Arg Ala Ser
Asp Asp Ala Ala Gly Met Gly Val Ser Gly Lys Ile Asn Ala Gln
Ile Arg Gly Leu Ser Gln Ala Ser Arg Asn Thr Ser Lys Ala Ile
Asn Phe Ile Gln Thr Thr Glu Gly Asn Leu Asn Glu Val Glu Lys
Val Leu Val Arg Met Lys Glu Leu Ala Val Gln Ser Gly Asn Gly
Thr Tyr Ser Asp Ser Asp Arg Gly Ser Ile Gln Ile Glu Ile Glu
Gln Leu Thr Asp Glu Ile Asn Arg Ile Ala Asp Gln Ala Gln Tyr
Asn Gln Met His Met Leu Ser Asn Lys Ser Ala Ser Gln Asn Val
Lys Thr Ala Glu Glu Leu Gly Met Gln Pro Ala Lys Ile Asn Thr
Pro Ala Ser Leu Ser Gly Ser Gln Ala Ser Trp Thr Leu Arg Val
His Val Gly Ala Asn Gln Asp Glu Ala Ile Ala Val Asn Ile Tyr
Ser Ala Asn Val Ala Asn Leu Phe Ala Gly Glu Gly Ala Gln Ala
Ala Gln Ala Ala Pro Val Gln Glu Gly Ala Gln Glu Glu Gly Ala
Gln Gln Pro Thr Pro Ala Thr Ala Pro Thr Gln Gly Gly Val Asn
Ser Pro Val Asn Val Thr Thr Thr Val Asp Ala Asn Thr Ser Leu
Ala Lys Ile Glu Asn Ala Ile Arg Met Ile Ser Asp Gln Arg Ala
Asn Leu Gly Ala Phe Gln Asn Arg Leu Glu Ser Ile Lys Asn Ser
Thr Glu Tyr Ala Ile Glu Asn Leu Lys Ala Ser Tyr Ala Gln Ile
Lys Asp Ala Thr Met Thr Asp Glu Val Val Ala Ala Thr Thr Asn
Ser Ile Leu Thr Gln Ser Ala Met Ala Met Ile Ala Gln Ala Asn
Gln Val Pro Gln Tyr Val Leu Ser Leu Leu Arg
 ou de la séquence partielle de cette protéine présentent une délétion de 20-25 aminoacides au terminus N de la protéine, ou
d) elle présente la séquence d'aminoacides 
Met Lys Lys Met Leu Leu Ile Phe Ser Phe Phe Leu Val Phe Leu
Asn Gly Phe Pro Leu Asn Ala Arg Glu Val Asp Lys Glu Lys Leu
Lys Asp Phe Val Asn Met Asp Leu Glu Phe Val Asn Tyr Lys Gly
Pro Tyr Asp Ser Thr Asn Thr Tyr Glu Gln Ile Val Gly Ile Gly
Glu Phe Leu Ala Arg Pro Leu Ile Asn Ser Asn Ser Asn Ser Ser
Tyr Tyr Gly Lys Tyr Phe Val Asn Arg Phe Ile Asp Asp Gln Asp
Lys Lys Ala Ser Val Asp Ile Phe Ser Ile Gly Ser Lys Ser Glu
Leu Asp Ser Ile Leu Asn Leu Arg Arg Ile Leu Thr Gly Tyr Leu
Ile Lys Ser Phe Asp Tyr Glu Arg Ser Ser Ala Glu Leu Ile Ala
Lys Ala Ile Thr Ile Tyr Asn Ala Val Tyr Arg Gly Asp Leu Asp
Tyr Tyr Lys Glu Phe Tyr Ile Glu Ala Ser Leu Lys Ser Leu Thr
Lys Glu Asn Ala Gly Leu Ser Arg Val Tyr Ser Gln Trp Ala Gly
Lys Thr Gln Ile Phe Ile Pro Leu Lys Lys Asn Ile Leu Ser Gly
Asn Val Glu Ser Asp Ile Asp Ile Asp Ser Leu Val Thr Asp Lys
Val Val Ala Ala Leu Leu Ser Glu Asn Glu Ser Gly Val Asn Phe
Ala Arg Asp Ile Thr Asp Ile Gln Gly Glu Thr His Lys Ala Asp
Gln Asp Lys Ile Asp Ile Glu Leu Asp Asn Phe His Glu Ser Asp
Ser Asn Ile Thr Glu Thr Ile Glu Asn Leu Arg Asp Gln Leu Glu
Lys Ala Thr Asp Glu Glu His Lys Lys Glu Ile Glu Ser Gln Val
Asp Ala Lys Lys Lys Gln Lys Glu Glu Leu Asp Lys Lys Ala Ile
Asp Leu Asp Lys Ala Gln Gln Lys Leu Asp Phe Ala Glu Asp Asn
Leu Asp Ile Gln Arg Asp Thr Val Arg Glu Lys Leu Gln Glu Asn
Ile Asn Glu Thr Asn Lys Glu Lys Asn Leu Pro Lys Pro Gly Asp
Val Ser Ser Pro Lys Val Asp Lys Gln Leu Gln Ile Lys Glu Ser
Leu Glu Asp Leu Gln Glu Gln Leu Lys Glu Ala Ser Asp Glu Asn
Gln Lys Arg Glu Ile Glu Lys Gln Ile Glu Ile Lys Lys Asn Asp
Glu Glu Leu Phe Lys Asn Lys Asp His Lys Ala Leu Asp Leu Lys
Gln Glu Leu Asn Ser Lys Ala Ser Ser Lys Glu Lys Ile Glu Gly
Glu Glu Glu Asp Lys Glu Leu Asp Ser Lys Lys Asn Leu Glu Pro
Val Ser Glu Ala Asp Lys Val Asp Lys Ile Ser Lys Ser Asn Asn
Asn Glu Val Ser Lys Leu Ser Pro Leu Asp Glu Pro Ser Tyr Ser
Asp Ile Asp Ser Lys Glu Gly Val Asp Asn Lys Asp Val Asp Leu
Gln Lys Thr Lys Pro Gln Val Glu Ser Gln Pro Thr Ser Leu Asn
Glu Asp Leu Ile Asp Val Ser Ile Asp Ser Ser Asn Pro Val Phe
Leu Glu Val Ile Asp Pro Ile Thr Asn Leu Gly Thr Leu Gln Leu
Ile Asp Leu Asn Thr Gly Val Arg Leu Lys Glu Ser Ala Gln Gln
Gly Ile Gln Arg Tyr Gly Ile Tyr Glu Arg Glu Lys Asp Leu Val
Val Ile Lys Ile Asp Ser Gly Lys Ala Lys Leu Gln Ile Leu Asp
Lys Leu Glu Asn Leu Lys Val Ile Ser Glu Ser Asn Phe Glu Ile
Asn Lys Asn Ser Ser Leu Tyr Val Asp Ser Arg Met Ile Leu Val
Val Val Lys Asp Asp Ser Asn Ala Trp Arg Leu Ala Lys Phe Ser
Pro Lys Asn Leu Asp Glu Phe Ile Leu Ser Glu Asn Lys Ile Leu
Pro Phe Thr Ser Phe Ala Val Arg Lys Asn Phe Ile Tyr Leu Gln
Asp Glu Leu Lys Ser Leu Val Thr Leu Asp Val Asn Thr Leu Lys
Lys Val Lys
ou une séquence partielle de celle-ci d'au moins 10 aminoacides.

2. Protéine immunologiquement active suivant la revendication 1, **caractérisée en ce qu'**on peut la préparer en recourant à l'emploi d'ADN isolé de Borrelia burgdorferi.

3. Protéine immunologiquement active suivant la revendication 2, **caractérisée en ce qu'**on peut la préparer en recourant à l'emploi d'ADN isolé de Borrelia burgdorferi (DSM-n° 5662).

4. Trousse d'essai pour la détermination d'anti-corps contre des souches de Borrelia, **caractérisée en ce qu'**elle contient au moins une protéine immunologiquement active suivant l'une des revendications 1 à 3, qui est susceptible de réagir avec des anticorps présents dans le liquide examiné et **en ce qu'**elle présente au moins un composant indicateur qui permet la détermination de complexes constitués d'anticorps et de la protéine immunologiquement active.

5. Trousse d'essai suivant la revendication 4, **caractérisée en ce qu'**elle contient de 2 à 4 protéines immunologiquement actives suivant les revendications 1 à 3.

6. Trousse d'essai suivant l'une des revendications 4 ou 5, **caractérisée en ce que** le composant indicateur est un anticorps dirigé contre l'anticorps à déterminer, qui présente un marquage.

7. Trousse d'essai suivant la revendication 6, **caractérisée en ce que** le marquage se compose d'un isotope radioactif.

8. Trousse d'essai suivant l'une des revendications 4 à 6, **caractérisée en ce que** le marquage se compose d'une enzyme qui peut catalyser une réaction chromogène.

9. Trousse d'essai suivant les revendications 4 ou 5, **caractérisée en ce que** la protéine immunologiquement active, ou un anticorps monoclonal dirigé contre cette protéine, ont subi une biotinylation et **en ce que** le composant indicateur est de l'avidine ou de la streptavidine avec une enzyme qui y liée par covalence, plus particulièrement une peroxydase.

10. Trousse d'essai suivant l'une des revendications 4 à 6, **caractérisée en ce qu'**elle est une trousse d'essai-ELISA.

11. Trousse d'essai suivant la revendication 10, **caractérisée en ce qu'**au moins une protéine immunologiquement active suivant l'une des revendications 1 à 3 est couplée à des plaques de microtitrage et le composant indicateur se compose d'une immunoglobuline anti-humaine, plus particulièrement des anticorps IgG et/ou IgM, auxquels est couplée une enzyme qui catalyse une réaction chromogène.

12. Utilisation de protéines immunologiquement actives suivant l'une des revendications 1 à 3 pour la préparation de vaccins conférant une protection contre des infections par des bactéries Borrelia, de préférence, des souches de Borrelia burgdorferi.

## Revendications (Revendications pour l'(les) Etat(s) contractant(s) suivant(s): ES)

1. Procédé de préparation d'une protéine immunologiquement active de Borrelia burgdorferi qui se présente sous une forme dépourvue d'autres protéines issues de Borrelia burgdorferi, et
a) qui présente la séquence d'aminoacides 
Met Lys Lys Asn Thr Leu Thr Ala Ile Leu Met Thr Leu Phe Leu
Phe Ile Ser Cys Asn Asn Ser Gly Lys Val Gly Ile Leu Thr Ser
Thr Asn Pro Ala Asp Glu Ser Ala Lys Gly Pro Asn Leu Thr Glu
Ile Ser Lys Lys Ile Thr Asp Ser Asn Ala Phe Val Leu Ala Val
Lys Glu Val Glu Thr Leu Val Leu Ser Ile Asp Glu Leu Ala Lys
Lys Ala Ile Gly Gln Lys Ile Asp Asn Asn Asn Gly Leu Ala Ala
Leu Asn Asn Gln Asn Gly Ser Leu Leu Ala Gly Ala Tyr Ala Ile
Ser Thr Leu Ile Thr Glu Lys Leu Ser Lys Leu Lys Asn Leu Glu
Glu Leu Lys Thr Glu Ile Ala Lys Ala Lys Lys Cys Ser Glu Glu
Phe Thr Asn Lys Leu Lys Ser Gly His Ala Asp Leu Gly Lys Gln
Asp Ala Thr Asp Asp His Ala Lys Ala Ala Ile Leu Lys Thr His
Ala Thr Thr Asp Lys Gly Ala Lys Glu Phe Lys Asp Leu Phe Glu
Ser Val Glu Gly Leu Leu Lys Ala Ala Gln Val Ala Leu Thr Asn
Ser Val Lys Glu Leu Thr Ser Pro Val Val Ala Glu Ser Pro Lys
Lys Pro
 ou une séquence partielle de celle-ci d'au moins 10 aminoacides
ou
b) qui présente la séquence d'aminoacides 
Met Lys Lys Asn Thr Leu Ser Ala Ile Leu Met Thr Leu Phe Leu
Phe Ile Ser Cys Asn Asn Ser Gly Lys Asp Gly Asn Thr Ser Ala
Asn Ser Ala Asp Glu Ser Val Lys Gly Pro Asn Leu Thr Glu Ile
Ser Lys Lys Ile Thr Asp Ser Asn Ala Val Leu Leu Ala Val Lys
Glu Val Glu Ala Leu Leu Ser Ser Ile Asp Glu Ile Ala Ala Lys
Ala Ile Gly Lys Lys Ile His Gln Asn Asn Gly Leu Asp Thr Glu
Asn Asn His Asn Gly Ser Leu Leu Ala Gly Ala Tyr Ala Ile Ser
Thr Leu Ile Lys Gln Lys Leu Asp Gly Leu Lys Asn Glu Gly Leu
Lys Glu Lys Ile Asp Ala Ala Lys Lys Cys Ser Glu Thr Phe Thr
Asn Lys Leu Lys Glu Lys His Thr Asp Leu Gly Lys Glu Gly Val
Thr Asp Ala Asp Ala Lys Glu Ala Ile Leu Lys Thr Asn Gly Thr
Lys Thr Lys Gly Ala Glu Glu Leu Gly Lys Leu Phe Glu Ser Val
Glu Val Leu Ser Lys Ala Ala Lys Glu Met Leu Ala Asn Ser Val
Lys Glu Leu Thr Ser Pro Val Val Ala Glu Ser Pro Lys Lys Pro
 ou une séquence partielle de celle-ci d'au moins 10 aminoacides
ou
c) qui est constituée de 
Met Arg Gly Ser Ile Met Ile Ile Asn His Asn Thr Ser Ala Ile
Asn Ala Ser Arg Asn Asn Ala Ile Asn Ala Ala Asn Leu Ser Lys
Thr Gln Glu Lys Leu Ser Ser Asn Tyr Arg Ile Asn Arg Ala Ser
Asp Asp Ala Ala Gly Met Gly Val Ser Gly Lys Ile Asn Ala Gln
Ile Arg Gly Leu Ser Gln Ala Ser Arg Asn Thr Ser Lys Ala Ile
Asn Phe Ile Gln Thr Thr Glu Gly Asn Leu Asn Glu Val Glu Lys
Val Leu Val Arg Met Lys Glu Leu Ala Val Gln Ser Gly Asn Gly
Thr Tyr Ser Asp Ser Asp Arg Gly Ser Ile Gln Ile Glu Ile Glu
Gln Leu Thr Asp Glu Ile Asn Arg Ile Ala Asp Gln Ala Gln Tyr
Asn Gln Met His Met Leu Ser Asn Lys Ser Ala Ser Gln Asn Val
Lys Thr Ala Glu Glu Leu Gly Met Gln Pro Ala Lys Ile Asn Thr
Pro Ala Ser Leu Ser Gly Ser Gln Ala Ser Trp Thr Leu Arg Val
His Val Gly Ala Asn Gln Asp Glu Ala Ile Ala Val Asn Ile Tyr
Ser Ala Asn Val Ala Asn Leu Phe Ala Gly Glu Gly Ala Gln Ala
Ala Gln Ala Ala Pro Val Gln Glu Gly Ala Gln Glu Glu Gly Ala
Gln Gln Pro Thr Pro Ala Thr Ala Pro Thr Gln Gly Gly Val Asn
Ser Pro Val Asn Val Thr Thr Thr Val Asp Ala Asn Thr Ser Leu
Ala Lys Ile Glu Asn Ala Ile Arg Met Ile Ser Asp Gln Arg Ala
Asn Leu Gly Ala Phe Gln Asn Arg Leu Glu Ser Ile Lys Asn Ser
Thr Glu Tyr Ala Ile Glu Asn Leu Lys Ala Ser Tyr Ala Gln Ile
Lys Asp Ala Thr Met Thr Asp Glu Val Val Ala Ala Thr Thr Asn
Ser Ile Leu Thr Gln Ser Ala Met Ala Met Ile Ala Gln Ala Asn
Gln Val Pro Gln Tyr Val Leu Ser Leu Leu Arg
 ou de la séquence partielle de cette protéine présentant une délétion de 20-25 aminoacides au terminus N de la protéine, ou
d) qui présente la séquence d'aminoacides 
Met Lys Lys Met Leu Leu Ile Phe Ser Phe Phe Leu Val Phe Leu
Asn Gly Phe Pro Leu Asn Ala Arg Glu Val Asp Lys Glu Lys Leu
Lys Asp Phe Val Asn Met Asp Leu Glu Phe Val Asn Tyr Lys Gly
Pro Tyr Asp Ser Thr Asn Thr Tyr Glu Gln Ile Val Gly Ile Gly
Glu Phe Leu Ala Arg Pro Leu Ile Asn Ser Asn Ser Asn Ser Ser
Tyr Tyr Gly Lys Tyr Phe Val Asn Arg Phe Ile Asp Asp Gln Asp
Lys Lys Ala Ser Val Asp Ile Phe Ser Ile Gly Ser Lys Ser Glu
Leu Asp Ser Ile Leu Asn Leu Arg Arg Ile Leu Thr Gly Tyr Leu
Ile Lys Ser Phe Asp Tyr Glu Arg Ser Ser Ala Glu Leu Ile Ala
Lys Ala Ile Thr Ile Tyr Asn Ala Val Tyr Arg Gly Asp Leu Asp
Tyr Tyr Lys Glu Phe Tyr Ile Glu Ala Ser Leu Lys Ser Leu Thr
Lys Glu Asn Ala Gly Leu Ser Arg Val Tyr Ser Gln Trp Ala Gly
Lys Thr Gln Ile Phe Ile Pro Leu Lys Lys Asn Ile Leu Ser Gly
Asn Val Glu Ser Asp Ile Asp Ile Asp Ser Leu Val Thr Asp Lys
Val Val Ala Ala Leu Leu Ser Glu Asn Glu Ser Gly Val Asn Phe
Ala Arg Asp Ile Thr Asp Ile Gln Gly Glu Thr His Lys Ala Asp
Gln Asp Lys Ile Asp Ile Glu Leu Asp Asn Phe His Glu Ser Asp
Ser Asn Ile Thr Glu Thr Ile Glu Asn Leu Arg Asp Gln Leu Glu
Lys Ala Thr Asp Glu Glu His Lys Lys Glu Ile Glu Ser Gln Val
Asp Ala Lys Lys Lys Gln Lys Glu Glu Leu Asp Lys Lys Ala Ile
Asp Leu Asp Lys Ala Gln Gln Lys Leu Asp Phe Ala Glu Asp Asn
Leu Asp Ile Gln Arg Asp Thr Val Arg Glu Lys Leu Gln Glu Asn
Ile Asn Glu Thr Asn Lys Glu Lys Asn Leu Pro Lys Pro Gly Asp
Val Ser Ser Pro Lys Val Asp Lys Gln Leu Gln Ile Lys Glu Ser
Leu Glu Asp Leu Gln Glu Gln Leu Lys Glu Ala Ser Asp Glu Asn
Gln Lys Arg Glu Ile Glu Lys Gln Ile Glu Ile Lys Lys Asn Asp
Glu Glu Leu Phe Lys Asn Lys Asp His Lys Ala Leu Asp Leu Lys
Gln Glu Leu Asn Ser Lys Ala Ser Ser Lys Glu Lys Ile Glu Gly
Glu Glu Glu Asp Lys Glu Leu Asp Ser Lys Lys Asn Leu Glu Pro
Val Ser Glu Ala Asp Lys Val Asp Lys Ile Ser Lys Ser Asn Asn
Asn Glu Val Ser Lys Leu Ser Pro Leu Asp Glu Pro Ser Tyr Ser
Asp Ile Asp Ser Lys Glu Gly Val Asp Asn Lys Asp Val Asp Leu
Gln Lys Thr Lys Pro Gln Val Glu Ser Gln Pro Thr Ser Leu Asn
Glu Asp Leu Ile Asp Val Ser Ile Asp Ser Ser Asn Pro Val Phe
Leu Glu Val Ile Asp Pro Ile Thr Asn Leu Gly Thr Leu Gln Leu
Ile Asp Leu Asn Thr Gly Val Arg Leu Lys Glu Ser Ala Gln Gln
Gly Ile Gln Arg Tyr Gly Ile Tyr Glu Arg Glu Lys Asp Leu Val
Val Ile Lys Ile Asp Ser Gly Lys Ala Lys Leu Gln Ile Leu Asp
Lys Leu Glu Asn Leu Lys Val Ile Ser Glu Ser Asn Phe Glu Ile
Asn Lys Asn Ser Ser Leu Tyr Val Asp Ser Arg Met Ile Leu Val
Val Val Lys Asp Asp Ser Asn Ala Trp Arg Leu Ala Lys Phe Ser
Pro Lys Asn Leu Asp Glu Phe Ile Leu Ser Glu Asn Lys Ile Leu
Pro Phe Thr Ser Phe Ala Val Arg Lys Asn Phe Ile Tyr Leu Gln
Asp Glu Leu Lys Ser Leu Val Thr Leu Asp Val Asn Thr Leu Lys
Lys Val Lys
ou une séquence partielle de celle-ci d'au moins 10 aminoacides,
**caractérisé en ce qu'**elle est préparée par la technique génétique.

2. Procédé suivant la revendication 1, **caractérisé en ce que** la protéine immunologiquement active peut être préparée en recourant à l'emploi d'ADN isolé de Borrelia burgdorferi.

3. Procédé suivant la revendication 2, **caractérisé en ce que** la protéine immunologiquement active peut être préparée en recourant à l'emploi d'ADN isolé de Borrelia burgdorferi (DSM-n° 5662).

4. Trousse d'essai pour la détermination d'anti-corps contre des souches de Borrelia, **caractérisée en ce qu'**elle contient au moins une protéine immunologiquement active qui est préparable suivant l'une des revendications 1 à 3 et qui est susceptible de réagir avec des anticorps présents dans le liquide examiné et **en ce qu'**elle présente au moins un composant indicateur qui permet la détermination de complexes constitués d'anticorps et de la protéine immunologiquement active.

5. Trousse d'essai suivant la revendication 4, **caractérisée en ce qu'**elle contient de 2 à 4 protéines immunologiquement actives préparables suivant les revendications 1 à 3.

6. Trousse d'essai suivant l'une des revendications 4 ou 5, **caractérisée en ce que** le composant indicateur est un anticorps dirigé contre l'anticorps à déterminer, qui présente un marquage.

7. Trousse d'essai suivant la revendication 6, **caractérisée en ce que** le marquage se compose d'un isotope radioactif.

8. Trousse d'essai suivant l'une des revendications 4 à 6, **caractérisée en ce que** le marquage se compose d'une enzyme qui peut catalyser une réaction chromogène.

9. Trousse d'essai suivant les revendications 4 ou 5, **caractérisée en ce que** la protéine immunologiquement active, ou un anticorps monoclonal dirigé contre cette protéine, ont subi une biotinylation et **en ce que** le composant indicateur est de l'avidine ou de la streptavidine avec une enzyme qui y liée par covalence, plus particulièrement une peroxydase.

10. Trousse d'essai suivant l'une des revendications 4 à 6, **caractérisée en ce qu'**elle est une trousse d'essai-ELISA.

11. Trousse d'essai suivant la revendication 10, **caractérisée en ce qu'**au moins une protéine immunologiquement active suivant l'une des revendications 1 à 3 est couplée à des plaques de microtitrage et le composant indicateur se compose d'une immunoglobuline anti-humaine, plus particulièrement des anticorps IgG et/ou IgM, auxquels est couplée une enzyme qui catalyse une réaction chromogène.

12. Procédé de préparation de vaccins conférant une protection contre des infections par des bactéries Borrelia de préférence, des souches de Borrelia burgdorferi, **caractérisé en ce qu'**on utilise des protéines immunologiquement actives qui peuvent être préparées selon l'une des revendications 1 à 3.
